# EUROPEAN PATENT SPECIFICATION

(11) **EP 1 412 383 B1**
(45) Date of publication and mention of the grant of the patent: **15.11.2006**
(21) Application number: 02764797.3
(22) Date of filing: 26.07.2002
(51) Int. Cl.: C07K 14/595, C07K 14/655, C07C 237/12, C07F 9/38, A61K 38/04, A61K 51/08, G01N 33/68, C07F 9/572

(54) **PEPTIDE CONJUGATES, THEIR DERIVATIVES WITH METAL COMPLEXES AND USE THEREOF FOR MAGNETIC RESONANCE IMAGING (MRI)**
PEPTIDKONJUGATE, DEREN DERIVATE MIT METALLISCHEN KOMPLEXEN UND DEREN VERWENDUNG ZUR BILDGEBENDEN MAGNETISCHEN RESONANZ
CONJUGUES PEPTIDIQUES, LEURS DERIVES A COMPLEXES METALLIQUES ET LEUR UTILISATION DANS L'IMAGERIE PAR RESONANCE MAGNETIQUE (IRM)

(30) Priority: 03.08.2001 IT MI20011708
(43) Date of publication of application: 28.04.2004
(73) Proprietor: BRACCO IMAGING S.p.A., 20134 Milano (IT)
(72) Inventor: AIME, Silvio, I-20134 Milano (IT); GIANOLIO, Eliana, I-20134 Milano (IT); MORELLI, Giancarlo, I-20134 Milano (IT); PEDONE, Carlo, I-20134 Milano (IT); TESAURO, Diego, I-20134 Milano (IT); LATTUADA, Luciano, I-20134 Milano (IT); VISIGALLI, Massimo, I-20134 Milano (IT); ANELLI, Pier, Lucio, I-20134 Milano (IT)
(74) Representative: Minoja, Fabrizio
(86) International application number: PCT/EP2002/008382
(87) International publication number: WO 2003/014157

(56) References cited:
- EP-A- 0 515 313
- EP-A- 0 607 103
- EP-A- 0 714 911
- WO-A-00/30688
- WO-A-01/46207
- WO-A-90/12050
- WO-A-94/02506
- WO-A-98/41540
- L A BASS ET AL.: "Identification of the soluble in vivo metabolites of indium-111-diethylenetetraminepentaacetic acid-D-Phe-octreotide " BIOCONJUGATE CHEMISTRY, vol. 9, no. 2, 14 February 1998 (1998-02-14), pages 192-200, XP002216302 WASHINGTON US
- R M VALERIO ET AL.: "Multipin solid-phase synthesis of acyl 2,3-diaminopropionic acid oligomers" INTERNATIONAL JOURNAL OF PEPTIDE AND PROTEIN RESEARCH., vol. 47, 1996, pages 414-418, XP002233379 MUNKSGAARD, COPENHAGEN., DK ISSN: 0367-8377
- DATABASE CA [Online] CHEMICAL ABSTRACTS SERVICE, COLUMBUS, OHIO, US; ERTAY, T. ET AL: "New radiolabeled CCK-8 analogues [Tc-99m-GH-CCK-8 and Tc-99m-DTPA-CCK-8]: preparation and biodistribution studies in rats and rabbits" retrieved from STN Database accession no. 136:321340 CA XP002233380 & NUCLEAR MEDICINE AND BIOLOGY (2001), 28(6), 667-678 , 2001,

## Description

### DISCLOSURE

The present invention relates to a novel class of contrast agents for use in magnetic resonance imaging (MRI), to identify and locate primary human tumours and their metastases which over-express type CCK A and/or type B cholecystokinin receptors, and/or type SSTR 1-5 somatostatin receptors.

Medical diagnosis using magnetic resonance imaging, which is recognized as a powerful diagnostic procedure in clinical practice, mainly employs paramagnetic pharmaceutical compositions, preferably containing complex chelates of bi-trivalent paramagnetic metal ions with polyaminopolycarboxylic acids and/or their derivatives or analogues.

Some of them are at present in clinical use as contrast agents for M.R.I. (Gd-DTPA, N-methylglucamine salt of the gadolinium complex with diethylenetriaminopentaacetic acid, MAGNEVIST^{®}; Gd-DOTA, N-methylglucamine salt of the gadolinium complex with 1,4,7,10-tetraazacyclododecane-1,4,7,10-tetraacetic acid, DOTAREM^{®}; Gd-HPDO3A gadolinium complex with 10-(2-hydroxypropyl)-1,4,7,10-tetraazacyclododecane-1,4,7-triacetic acid, PROHANCE^{®}; Gd-DTPA-BMA, gadolinium complex with diethylenetriaminopentaacetic acid bis(methylamide), OMNISCAN^{®}).

Said contrast agents and the derivatives thereof are disclosed in EP 715 64, DE-A-3401052, EP 130 934, US 4,639,365, US 4,615,879, EP 65 728, EP 299 795, EP 230 893, WO8905802, EP 258 616, US 4,826,673. Other pertinent compounds are cited in EP-A-607103, WO-A-9012050 and WO-A-200030688.

The commercially available contrast agents listed above are designed for a wholly general use. In fact, after administration the MRI contrast agent is distributed in the extracellular spaces in different parts of the body prior to being excreted. In this sense they behave in a similar manner to iodinated compounds used in X ray medical diagnosis.

At present there is increasing need for contrast agents that are aimed at specific organs, a need which is not adequately met by the products currently on the market.

An early, efficient diagnosis is increasingly required in oncology and this can be obtained only using very sensitive diagnostic procedures, which are able to detect a pathology at its very early stages.

In this context, the use of suitably functionalized contrast agents able to selectively bind to the receptors over-expressed by tumour cells, would provide an extremely sensitive diagnosis suitable for oncologic investigations, particularly when using MRI techniques, which are recognizably sensitive.

Somatostatin is a tetradecapeptide produced by the hypothalamus that exerts a number of activities on central nervous system, pancreas and gastrointestinal tract. In particular, somatostatin inhibits the release of insulin and glucagone from pancreas, the release of growth hormone by the hypothalamus and reduces stomach gastric secretion.

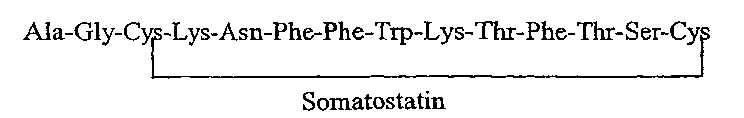
Ala-Gly-Cys-Lys-Asn-Phe-Phe-Trp-Lys-Thr-Phe-Thr-Ser-Cys
Somatostatin

Somatostatin was first characterized and disclosed by Guillemin et al. (see US 3904594) in 1975. This tetradecapeptide is characterized by a cyclic bond between two sulfhydryl groups of two cysteine residues respectively at the 3 and 14 positions of the peptide.

Somatostatin exerts its action through binding to specific receptors expressed also on the surface of tumour cells of pancreas, stomach and hypothalamus. Somatostatin binding to said receptors can therefore be exploited for diagnostic and therapeutical purposes.

Recently, at least five somatostatin receptor sub-classes have been evidenced, all of them belonging to the family of G-proteins associated receptors (see e.g. US 5,436,155; WO9714715; Biochemical and Biophysical Research Communications, 258, 689-694, 1999).

Furthermore, overexpression of somatostatin receptors has been evidenced in a high number of human tumours, such as primary or metastatic neuroendocrine tumours, pituitary, central nervous system, gastro-enteropancreatic, breast tumours as well as Hodgkin and non-Hodgkin lymphomas.

Albeit somatostatin has wide therapeutical applications, its low *in vivo* stability and fast degradation in the presence of peptidases limit its use. As a consequence, extensive studies have been carried out on some classes of peptide analogs of somatostatin, mostly Octreotide, Vapreotide and Lanreotide.

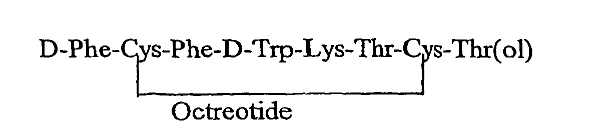
D-Phe-Cys-Phe-D-Trp-Lys-Thr-Cys-Thr(ol)
Octreotide

D-Phe-Cys-Phe-D-Trp-Lys-Val-Cys-Trp-NH2
Vapreotide

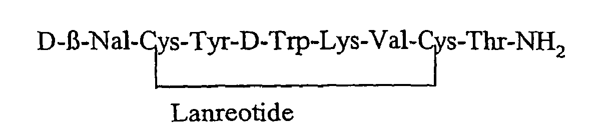
D-ß-Nal-Cys-Tyr-D-Trp-Lys-Val-Cys-Thr-NH2
Lanreotide

Octreotide, suitably functionalized, labelled with In-111 is already available (Octreoscan^{®}) for scintigraphic diagnosis of tumours (see e.g. J. Nucl. Med. 41, 1704-1713, 2000; Current Medicinal Chemistry, 7, 971-994, 2000).

Cholecystokinins (CCKs) are a family of peptide molecules whose biological action is performed as a hormone and a neurotransmitter. All the CCKs originate from a process of fragmentation which takes place on a pre-hormone consisting of 115 amino acid residues, followed by a post-translational process of alpha-amidation of the C-terminal phenylalanine residue and sometimes, sulfatation of the tyrosine residue contained in the C-terminal portion.

Cholecystokinins therefore exist in various molecular forms; the most important ones have a sequence of 58, 39, 33 or 8 amino acid residues, and they all have the same C-terminal sequence of 8 amino acid residues:

Asp-Tyr-Met-Gly-Trp-Met-Asp-Phe-amide.

in which the tyrosine residue can be sulfated. The octapeptide is known as CCK8.

The biological activity of cholecystokinin depends on the type of receptor with which it interacts. Two types of receptors are known: type A (from Alimentary) and type B (from Brain). In non-pathological conditions, type A receptor is present in the tissues of peripheral organs such as the stomach, gall bladder, intestine and pancreas. The most important physiological actions due to the interaction of the CCK peptide hormone with type A receptor are contraction of the gall bladder, secretion of pancreatic enzymes, regulation of secretion, and absorption into the gastrointestinal tract. Type B receptor is mainly present in the central nervous system, where the interaction with cholecystokinin causes analgesia, satiety and anxiety, and regulates the release of dopamine.

Both cholecystokinin receptors belong to the class of G-Protein Coupled Receptors (GPCRs), membrane receptors with seven transmembrane helices joined by intra- and extra-cellular loops with an extracellular N-terminal arm and an intracellular C-terminal part. Both receptors have high affinities for the various forms of cholecystokinin; however, type A receptor has a greater affinity for the sulfated forms of cholecystokinin, namely the ones which contain a sulfate group on the Tyr 27 residue, while type B receptor has a high affinity for the various forms of non-sulfated cholecystokinin and for gastrin. A series of peptide and non-peptide cholecystokinin-analog molecules with agonist or antagonist activity for type A and type B receptors are known (P. De Tullio, Current Medicinal Chemistry, 6, 433, 1999; F. Noble, Progress in Neurobiology, 58, 349, 1999). No pharmacological application has been found for any of the known molecules due to their low bioavailability and low solubility or high enzymatic degradation.

Cholecystokinin receptors are often over-expressed in various tumours. Type B receptor is frequently over-expressed in medullary thyroid tumours, small cell lung tumour, astrocytomas, ovarian stromal tumours and, to a lesser extent, in gastroenteral-pancreatic tumours (see e.g. WO9851337 and WO9835707), in breast, endometrium and ovary adenocarcinomas. On the other hand, Type A receptor is over-expressed in gastroenteral-pancreatic tumours, meningioma and some neuroblastomas.

Cholecystokinin receptors have recently been identified in both primary human tumours and metastases (J.C. Reubi, Cancer Research, 57, 1377, 1997, and WO9731657). The use of functional peptides labelled with radioactive metals such as ¹²⁵I (Biochemical Journal, 89, 114-123, 1963),¹¹¹In or ¹¹⁵In, used in nuclear medicine to visualise human tumours, is also described by this author. Said peptides are labelled by means of a single chelating unit which may be sufficient for radio-therapeutic or radio-diagnostic applications but would not be sufficient for MRI applications, in view of the low relaxivity obtainable by only one paramagnetic metal chelated by the single chelating moiety.

Particularly, among the tumours cited above, those characterized by the small size or slow growth of cells, are hardly detected with the conventional diagnostic techniques. Therefore, the medical profession needs simple diagnostic techniques, which are selective both *in vitro* and *in vivo* for one or more receptor types and allow through a suitable binding to localize and make diagnosis of the tumour.

Accordingly, the present invention relates to contrast agents for *in* vivo diagnostics, more particularly for MRI imaging, obtainable by conjugating peptides deriving from somatostatin or cholecystokinin with complex chelates with paramagnetic metal ions.

The present invention relates to compounds of general formula (I):

[A]ₜ[P]-[B]ᵥ (I)

wherein:
- B: represents
- A: represents the following chelating, groups: ethylenediaminotetraacetic acid (EDTA), diethylenetriaminopentaacetic acid (DTPA), 1,4,7,10-tetraazacyclododecane-1,4,7,10-tetraacetic acid (DOTA), 1,4,7,10-tetraazacyclododecane-1,4,7-triacetic acid (D03A), 10-(2-hydroxypropyl)-1,4,7,10-tetraazacyclododecane-1,4,7-triacetic acid (HPDO3A), 4-carboxy-5,8,11-tris(earboxymethyl)-1-phenyl-2-oxa-5,8,11-triazatridecan-13-oic acid (BOPTA), N-[2-[bis-(carboxymethyl)amino]-3-(4-ethoxyphenyl)propyl]-N-[2-[bis(carboxymethyl)amino]ethyl]glycine (EOB-DTPA), N,N-bis[2-(carboxymethyl)[(methylcarbamoyl)methyl]amino]ethyl]glycine (DTPA-BMA), 2-methyl-1,4,7,10-tetraazacyclododecane-1,4,7,10-tetraacetic acid (MCTA), (α,α',α",α"')-tetramethyl-1,4,7,10-tetraazacyclododecane-1,4,7,10-tetracetic acid (DOTMA); the residue of a polyaminophosphonic acid ligand and derivatives thereof, polyaminophosphinic acid and derivatives thereof, in particular ethylenedinitrilotetrakis(methylphosphonic) acid (EDTP); 1,4,7,10-tetraazacyclododecane-1,4,7,10-tetrakis[methylene(methylphosphonic)] acid and 1,4,7,10-tetraazacyclododecane-1,4,7,10-tetrakis[methylene(methylphosphinic)] acid; the residue of macrocyclic chelants such as texaphyrins, porphyrins, phthalocyanines. Further examples of residues A, disclosed in WO 01/46207, are represented by the following formulae:

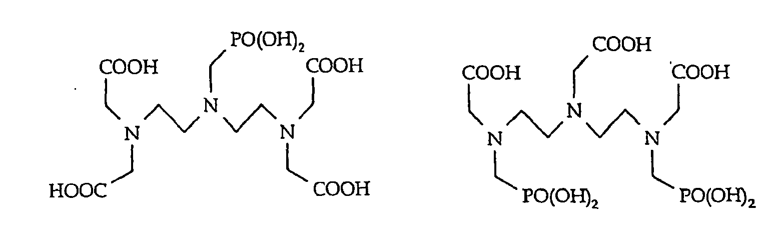

Furthermore, v is an integer of 1 to 5; t is an integer ranging from 3 to 15.

The invention also relates to the chelates of the compounds of general formula (I) with the bi- trivalent ions of metal elements having atomic numbers ranging from 21 to 29, 42, 44 or from 57 to 71, with paramagnetic metals, in particular with Fe, Cu, Cr, Eu, Gd, Tb, Dy, Ho, Er, Tm, Yb, Mn, as well as the salts thereof with physiologically compatible bases or acids.

Chelates of Fe(2+), Fe(3+), Cu(2+), Cr(2+), Cr(3+), Eu(3+), Gd(3+), Tb(3+), Dy(3+), Ho(3+), Er(3+), Yb(3+), Mn(2+) and Mn(3+) as well as the salts thereof with physiologically compatible bases or acids are preferred.

Particularly preferred are the complexes of bi- trivalent ions of Eu(3+), Gd(3+), Dy(3+), Mn(2+) and Mn(3+) as well as the salts thereof with physiologically compatible bases or acids.

P is a polylysme or a poly-L-lysine or a lys-β-Ala or Dap-β-Ala copolymer

Peptides **of formula (I)** selectively bind to one or more somatostatin receptors, known as SSTR type 1-5 receptors and/or subtype derivatives thereof and/or one or more cholecystokinin receptors and derivatives thereof, known as CCK-A and/or CCK-B receptors, which are over-expressed on a particular type of cells present in a tissue or organ of an animal or human being, *in vitro* and/or *in vivo.*

The compounds of the invention can be used as cholecystokinin (type A and/or B receptors) and/or somatostatin (SSTR type 1-5 receptors) agonists and/or antagonists and as MRI diagnostic agents for organs, tissues and cells of the human or animal body, for locating and detecting primary tumors and metastases thereof over-expressing said receptors.

The following peptides on used;

Gly-Glu-Tyr-Met-Gly-Trp-Met-Asp-Phe-NH₂

Gly-Asp-Tyr-Met-Gly-Trp-Leu-Asp-Phe-OH

Gly-Glu-Tyr(SO₃H)-Met-Gly-Trp-Leu-Asp-Phe-NH₂

Gly-Glu-Cys-Ala-D-Trp-Lys-Leu-Cys-Phe-OH

Gly-Ala-Cys-Gly-D-Trp-Lys-Leu-Cys-Phe-NH2

Gly-Ile-Cys-Ala-D-Trp-Lys-Glu-Cys-Lys-NH2

Said peptides are prepared using known methods, such as peptide synthetic methods in solid phase o in solution.

The term "any amino acid" reported above relates to the L and D

Examples of particularly preferred chelating groups A are shown in Scheme 1, in which they are reported in the form of "free acid", used in the complexation with the paramagnetic metal and in which Q means the preferred position for the covalent bond with the residue of the molecule.

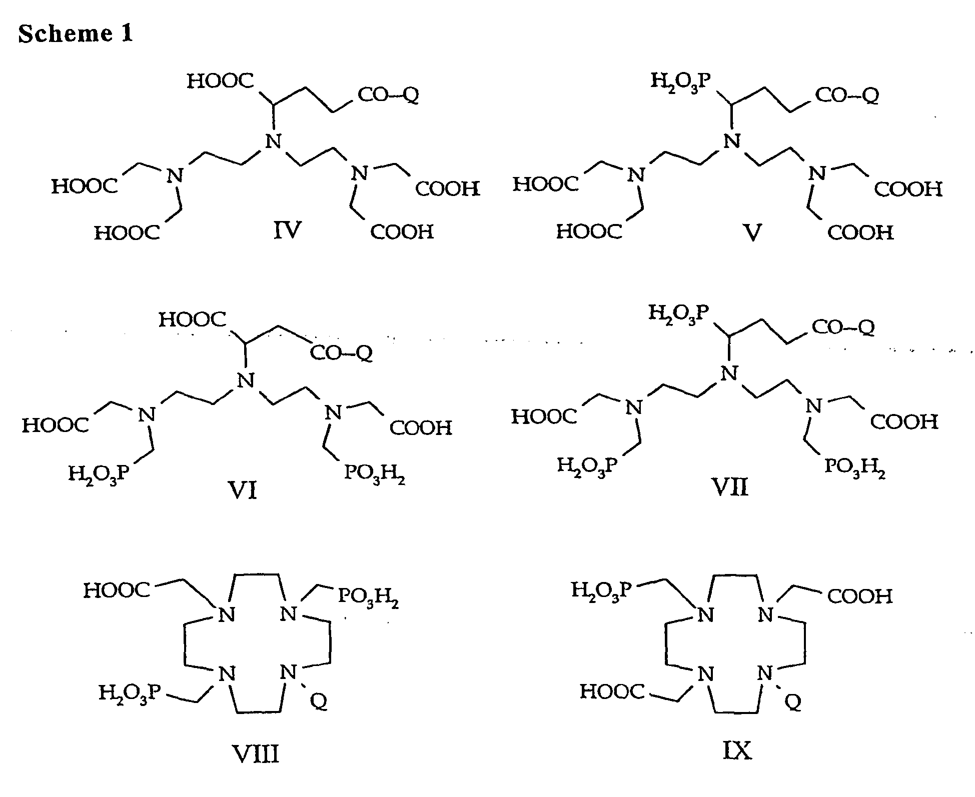

Acyclic chelating groups of formulae IV, V, VI and VII are more particularly preferred.

The number t of the chelating moieties A is preferably from 3 to 15, more preferably from 5 to 10.

Compounds of general formula (I) contain polymeric units P, which makes it possible, for example by changing their monomeric and/or oligomeric composition during preparation, to affect the chemical-physical characteristics thereof related for example to viscosity, solubility and intrinsic stability, as well as to provide a high number of metal chelating units in the molecule itself.

The polymer compounds used include, in fact, functional recurring units such as carboxylic, amino, hydroxy groups which can be conjugated with paramagnetic metal chelating agents A.

This type of compounds are particularly useful in MRI, as they allow to carry a comparatively high amount of paramagnetic metal ions per molecule unit to the receptor site, thereby inducing a corresponding specific signal of high intensity even at comparatively low molar dosages.

The polymer compound P can be selected from polypeptides such as polylysine, polyornithine, polyarginine, polyserine, polyglutamic acid, polyaspartic acid; polyamines such as polyallylamine, polyethyleneimine; polyacrylic acid derivatives such as poly-N-(2-aminoethyl)methacrylamide, poly-N-(2-hydroxypropyl)methacrylamide (HPMA); polyethyleneiminopolyacetic acid ester derivatives, poly(alkyleneoxides), alpha(polyamino)poly-(alkyleneoxides), poly(ethyleneoxide) (PEO), poly(propyleneoxide) (PPO), poly(butyleneoxide), polyoxypropylene glycol, polyoxyethylene glycol, polyoxyalkylene glycol, polyalkyl esters, polyalkylcyanoacrylates, polymethylcyanoacrylates, polyethylcyanoacrylates, polybutylcyanoacrylates, polysobutylcyanoacrylates. The meanings of P also includes, in addition to the already cited polymers, also copolymers obtained for example by a combination of those cited above. Polymer moieties P are functionalized to allow bonding with chelating units A and are conjugated with peptide compound B. Among units P conjugated with more chelating units A per molecule, those having good water solubility, as well as suitable viscosity and stability are preferred, in that said properties make them suitable for conjugation with peptide B.

Preferred polymer compounds are, for example, linear or branched polylysines, in particular poly-L-lysines, containing amino primary residues which make them suited for covalently bonding chelating units A directly to the free amino groups.

Further preferred P groups comprise co-polymers of two or more amino -acids, particularly of co-polymers of beta-alanine with lysine or 2,3-diaminopropionic acid (Dap), such as Lys-β-Ala and Dap-β-Ala copolymers.

Preferably, the polymeric or co-polymeric P moieties comprise from 2 to 15, more preferably from 3 to **10** monomeric or dimeric units.

When polymeric compound P is a repetition of dipeptide units, like β-Ala-Dap, the final compound already incorporating the chelating subunit is prepared by solid phase peptide synthesis using the Fmoc protocol and the subunits reported in scheme 2.

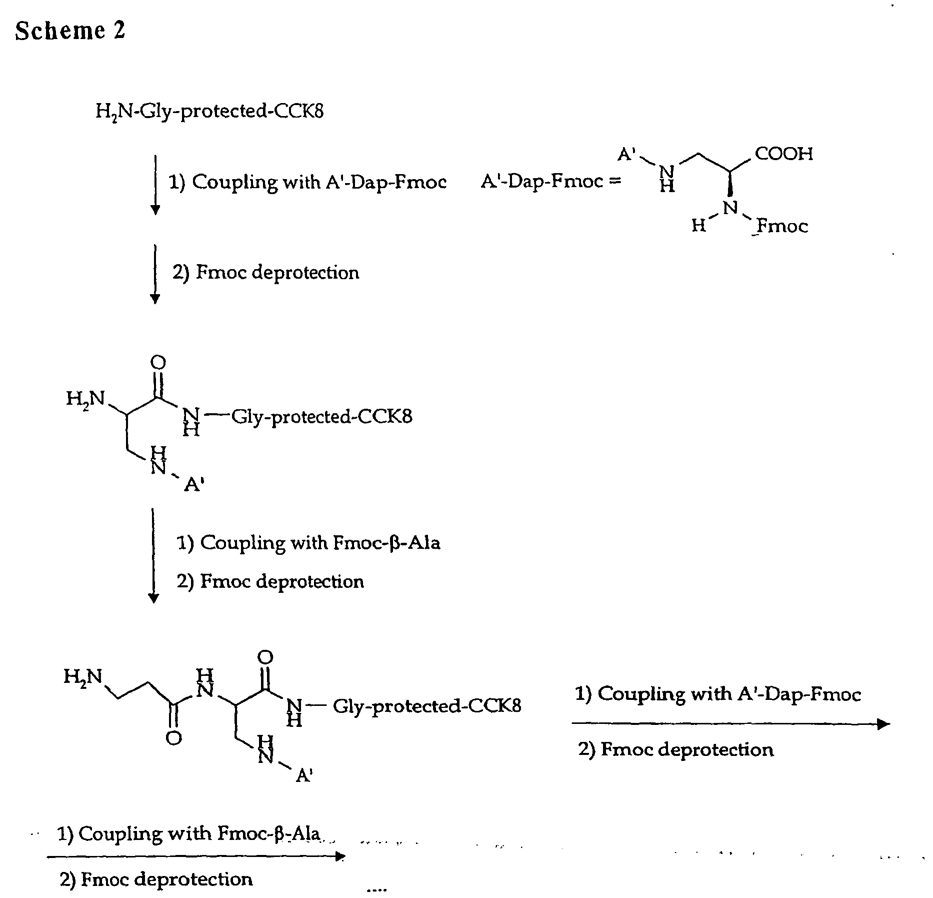

In this respect a particularly convenient building block is obtained by coupling of an α-Fmoc protected diaminoacid (like Dap) with the penta t-Bu ester of DTPA-GLU (see example 9). The functionalised Fmoc protected amino acid thus obtained offers the advantage of the simultaneous introduction of the chelating subunit while coupling the aminoacid with the Fmoc protocol. Said building blocks, which may be represented by the formula A'-Dap-Fmoc, wherein A' is a unit of formula A as defined above having the carboxy or phosphonic moieties suitably protected, Dap is a diamino-acid, specifically 2,3-diamino-propionic acid and Fmoc is (9H-fluoren-9-ylmethoxy)carbonyl, are useful intermediates and are a further object of the invention.

The complexes of compounds of formula (I) with the above defined metal ions are prepared according to known procedures, for example by reacting the compound containing one or more chelating units A with the oxide or halide of the selected metal ion.

More particularly, the process is carried out in water or in a suitable water/alcohol mixture at temperatures ranging from 25°C to 100°C, preferably from 25°C to 80°C.

If the resulting complex is insoluble in the reaction solvent, the solid product is filtered. If the complex is soluble, it can conveniently be recovered by evaporating off the solvent to a solid residue, for example with spray drying techniques after desalting the mixture by means of nanofiltration or chromatography through a suitable resin.

If the resulting complex contains free acid groups, such groups are salified by reaction with an organic or inorganic base. The resulting solution can subsequently be concentrated and the resulting complex salt can be suitably recovered by insolubilization or crystallization techniques.

The selection of the metal ion and of any neutralizing ions is closely related to the intended use of the complex.

Scheme 3 below shows the preparation of the compound **(DTPA-GLU)**_{**4**}**(Lys)**_{**2**}**Lys-Gly-Vapreotide** disclosed in Example 3 in which the conjugated peptide is cyclic and four DTPA-GLU units used as paramagnetic metal chelating groups are present in the molecule.

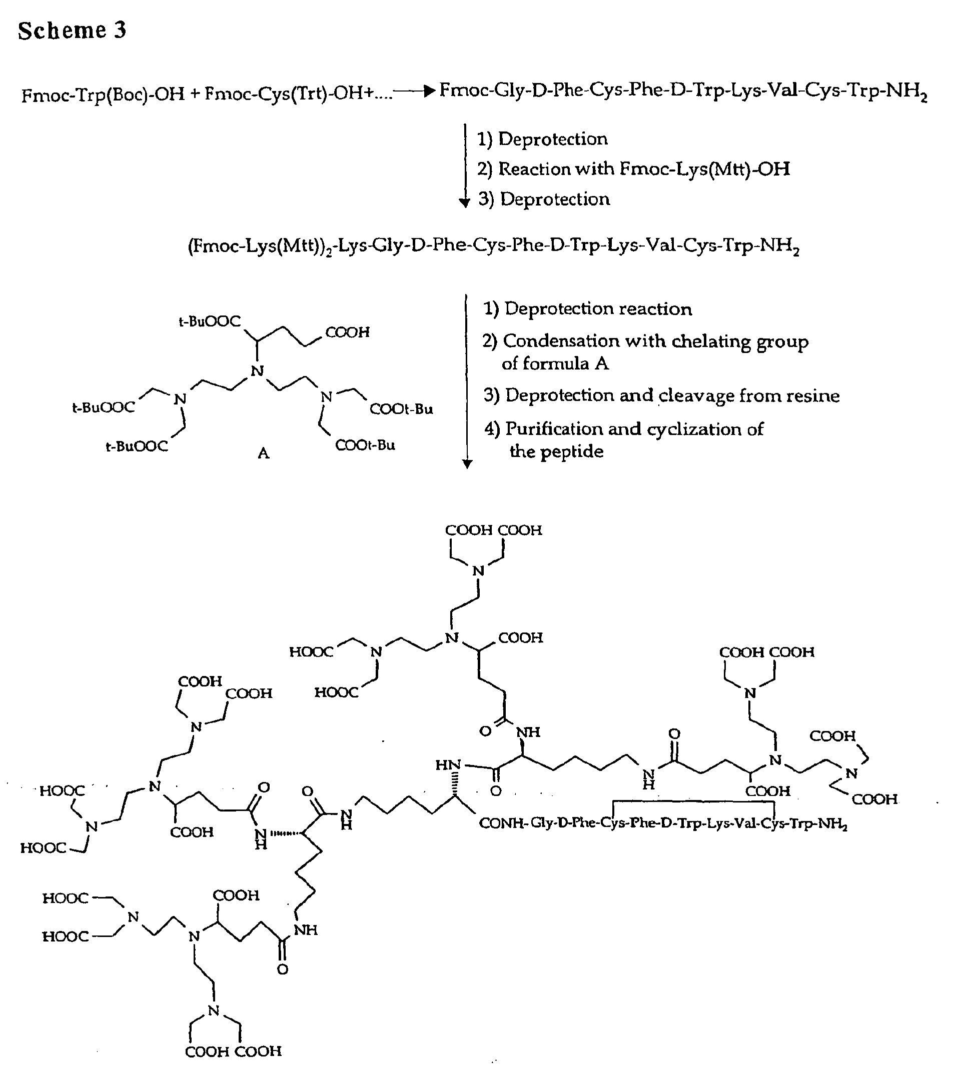

Examples of preferred compounds of formula (I) are reported below:

(DTPA-GLU)₄(Lys)₂Lys-Gly-CCK8

(DTPA-GLU)₃(Lys)₂Lys-Gly-CCK8

(DTPA-GLU)₄(Lys)₂Lys-Gly-Vapreotide

(DO3A-Ar)₄(Lys)₂Lys-Gly-Vapreotide

(Lys(DTPA-GLU)- βAla)₄Lys(DTPA-GLU)-Gly-CCK8

(Lys(DTPA-GLU)-βAla)₉ Lys(DTPA-GLU)-Gly-CCK8

(Lys(DTPA-GLU)-βAla)₄Lys(DTPA-GLU)-Gly-Vapreotide

(Lys(DTPA-GLU)-βAla)₉ Lys(DTPA-GLU)-Gly-Vapreotide

(Dap(DTPA-GLU)-βAla)₄-Dap(DTPA-GLU)-Gly-CCK8

(Dap(DTPA-GLU)-βAla)₉-Dap(DTPA-GLU)-Gly-CCK8

(Dap(DTPA-GLU)-βAla)₄-Dap(DTPA-GLU)-Vapreotide

(Dap(DTPA-GLU)-βAla)₉-Dap(DTPA-GLU)-Vapreotide

The compounds of the invention proved useful MRI contrast agents, in particular for imaging both tumour and non-tumour pathologies characterized by over-expression of cholecystokinin type CCK A and/or B and/or of somatostatin type SSTR 1-5 receptors.

The compounds of the invention are used both in *in vivo* and *in vitro* diagnostic methods, mainly in tumour cells, preferably in human tumour cells, imaging.

It should be stressed that binding may be followed by an internalization process.

The compounds of general formula (I) used *in vitro* are characterized by high relaxivity values compared with known, commercially available MRI contrast agents. The selective binding of the contrast agent to the receptors mentioned above, as well as the binding to macromolecules present in the surrounding medium and/or in the exo and/or endocellular system in which the compound is present and, for example, with plasma proteins, in the cellular or intracellular interstitial space contribute to attaining high relaxivity.

Interaction with macromolecules can take place, either at first with receptor binding of the contrast agent, or in subsequent phases, following interaction with P, B and A components present in the compounds of general formula (I). In this sense, interaction between contrast agent and macromolecules may also be affected by the presence in *vivo* of enzymes and/or microorganisms (e.g. hydrolases, peptidases, proteases, esterases and/or virus, bacteria, yeasts) which can cause the in situ modification of the contrast agent, thus affecting binding to the macromolecules themselves and to the receptors mentioned above.

Further examples of proteins featuring this phenomenon are, for example, human serum albumin (HSA; present both in plasma and in the interstitial space, although at a lower concentration), glutathione-S-transferase (GST or ligandine), fatty acid binding protein (FABP, Z-protein) and alpha 1-acid glycoprotein (AAC). The binding of contrast agent to macromolecule causes indeed a decrease in molecule flexibility in the bound state and therefore in the parameter known in MRI as rotation tumbling time or rotational correlation time, which induces an increase in relaxivity.

Interaction between contrast agent and, for example, protein components on the surface of (for example with the receptors cited above) or inside the cell, involves an increase in relaxation rate typical of MRI contrast, significantly increasing the tissue contrast which can be visualized in the MRI image.

Relaxivity data obtained by *in vitro* assays of compounds of formula (I) allow to envisage potential applications of these compounds as contrast agents in magnetic resonance for in *vivo* imaging cells, tissues or organs in which primary tumours or tumour pathologies or metastases are present.

A further object of the invention is the use of the compounds of general formula (I), suitably formulated, for imaging, for example, pancreatic and esophagus tumors and of other tumors over-expressing cholecystokinin type A receptors, small cells tumors of lung, colon, gastrointestinal tract, medullary thyroid carcinoma, astrocytomas, ovarian stromal tumours and other tumors over-expressing cholecystokinin type B receptors and moreover for imaging tumors (and metastases thereof) over-expressing somatostatin SSTR type 1-5 receptors such as: neuroendocrine, pituitary, central nervous system tumours, Hodgkin and non-Hodgkin lymphomas, breast and gastro-enteropancreatic tumours.

A further object of the present invention is an MRI procedure comprising the administration of a suitable amount of a paramagnetic complex according to the invention, for imaging and recording the image of, for example, an organ of the subject under investigation.

The compounds of the present invention are suitable for the oral or enteral administration.

For parenteral administration they can be preferentially formulated as sterile aqueous solutions or suspensions, whose pH can range from 6.0 to 8.5. These aqueous solutions or suspensions can be administered in concentrations ranging from 0.002 M to 1.0 M.

These formulations can be lyophilized and supplied as such, to be reconstituted just before the use. For the GI use or for injection to body cavities, these agents can be formulated as a solution or suspension containing suitable additives in order to, for example, control viscosity.

For the oral administration they can be formulated according to preparation methods routinely used in the pharmaceutical technique or as coated formulations to gain extra protection from the acid pH of stomach, thus inhibiting the release of the chelated metal ion.

The formulations of the invention comprise non covalent aggregates such as micelles, colloidal dispersions, oil-in-water emulsions, liposomes, nanocapsules, microbeads, inclusion compounds, solid lipid nanoparticles or pro-drugs, whose preparation requires functionalization with covalent bonds to suitable protective systems.

The preparation of liposomes which can contain a paramagnetic contrast agent is disclosed in EP 354 855, US 5 013 556, WO0011007, US 6 060 040, US 5 702 722, US 5 833 948, EP 759 785 which are incorporated herein by reference.

"Pro-drugs" herein means contrast agent precursors which provide the *in vivo* release of the product following administration and through a chemical and/or physiological process, such as enzymatic hydrolysis or change of pH.

Examples of pro-drugs are compounds in which a carboxylic function is substituted by an ester or amido group, an alcohol function derivatized to give an ester or ether, an amine function substituted with a suitable protective group suitable for the *in vivo* release of the product. A further example of pro-drug are compounds resulting from conjugation in form of PEG derivatives (see e.g. Biomaterials, 22, 405-417, 2001). Poly(ethylene glycol) (PEG), suitably conjugated to the peptide or to a functional group present in the compounds of general formula (I), induces a change in their characteristics while maintaining their biological properties, such as receptors selective binding, increased stability to enzymatic degradation by proteolytic enzymes, chemico-physical, biodistribution and solubility properties.

The pharmaceutical preparation may further contain conventional excipients, such as sweetening agents and/or flavours.

The formulations of the invention should anyway ensure a contrast agent concentration well below the toxicity limits and will optionally comprise both non covalent aggregates (micelles, SolidLipid Nanoparticles, liposomes, inclusion compounds) and pro-drugs which can be prepared through covalent bond to suitable protective systems (e.g. Pegylation).

Preferred cations of inorganic bases suitable for salifying the complexes of the invention comprise, in particular, alkali or alkaline-earth metal ions such as potassium, sodium, calcium, magnesium.

Preferred cations of organic bases comprise those of primary, secondary and tertiary amines, such as ethanolamine, diethanolamine, morpholine, glucamine, N-methylglucamine, N,N-dimethylglucamine.

Preferred anions of inorganic acids suitable for salifying the chelated complexes of the invention comprise, in particular, the ions of halo acids such as chlorides, bromides, iodides or other ions such as sulfate.

Preferred anions of organic acids for the above mentioned purposes comprise those of acids conventionally used in pharmaceutical technique for the salification of basic substances, such as acetate, succinate, citrate, fumarate, maleate, oxalate.

Preferred cations and anions of amino acids comprise, for example, those of taurine, glycine, lysine, arginine or ornithine or of aspartic and glutamic acids.

The following examples further illustrate the invention.

### Experimental section

Benzotriazole-1-yl-oxy-tris-pyrrolidino phosphonium hexafluorophosphate (PyBop), 1-hydroxybenzotriazole (HOBt), all the Fmoc-amino acid derivatives (Fmoc = 9-flurenylmethyloxycarbonyl) and the Rink amide MBHA resin were purchased from Calbiochem-Novabiochem (Laufelfingen, CH). [O-(7-azobenzotriazol-yl)-1,1,3,3-tetramethyluronium] hexafluorophosphate (HATU) was purchased from Applied Biosystem. N,N-Bis[2-[bis[2-(1,1-dimethylethoxy)-2-oxoethyl]amino]ethyl]-L-glutamic acid 1-(1,1-dimethylethyl) ester (the protected DTPA-GLU), was prepared as previously described. All other chemicals were obtained by Aldrich (Milwaukee, WI, USA) and were used without further purification unless otherwise stated.

Solid-phase peptide synthesis was performed on a fully automated synthesizer Shimadzu SPPS-8 (Kyoto, Japan) and ABI Perseptive Biosystem 433. Analytical RP-HPLC runs were carried out on a Shimadzu model 10A-LC apparatus using a Phenomenex (Torrance, CA, USA) C₁₈ column, 4.6x250 mm, eluted with H₂O/0.1% trifluoroacetic acid (TFA) (A) and CH₃CN/0.1% TFA (B) linear gradients from 5% to 70% B over 30 min at 1 ml/min flow rate. Preparative RP-HPLC runs were carried out on a Waters (Milford, MA, USA) Delta Prep 4000 instrument equipped with a UV Lambda-Max model 481 detector using a Vydac (Hesperia, CA, USA) C₁₈ column, 22x250 mm. A linear gradient from 20% to 80% B over 40 min at 20 mL/min flow rate was used.

Mass spectra were obtained on a Maldi-Tof Vojager-DE (Perseptive Biosystem, Foster City, CA, USA) apparatus.

### Example 1

### Synthesis of the gadolinium complex of (DTPA-GLU)₄(Lys)₂Lys-Gly-CCK8

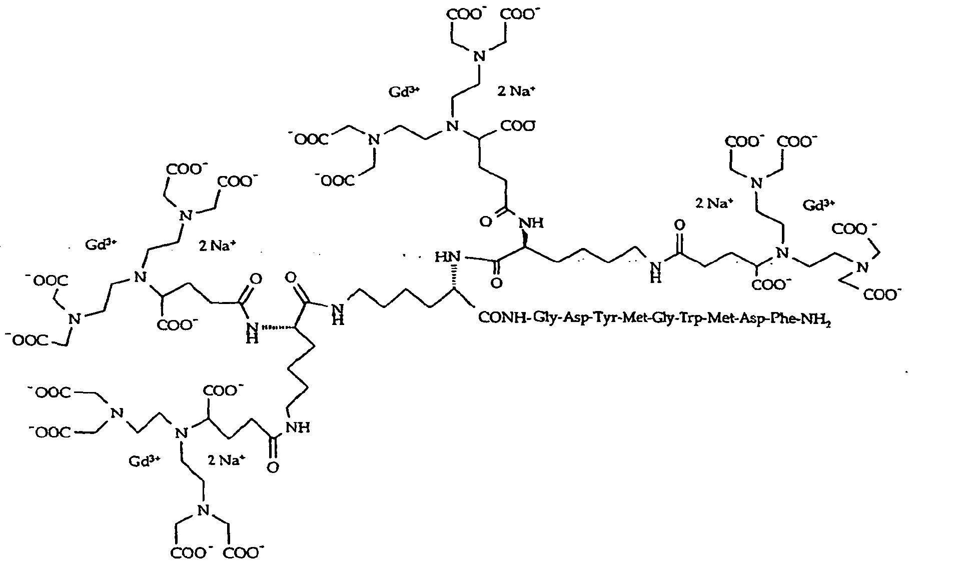

### A) Synthesis of (DTPA-GLU)₄(Lys)₂Lys-Gly-CCK8

Gly-CCK8 (sequence: H-Gly-Asp-Tyr-Met-Gly-Trp-Met-Asp-Phe-NH₂) synthesis was carried out in solid phase under standard conditions using the Fmoc protocol. The Rink-amide MBHA resin (0.54 mmol/g, 54 µmol scale, 0.100 g) was used. Double couplings were performed, adding each time four equivalents of N-protected amino acids activated by PyBop and HOBt and eight equivalents of *N,N*-diisopropylethylamine (DIPEA) in DMF, and stirring for 60 min. When the peptide Gly-CCK8 synthesis was complete, the resin was transferred into a vessel for Fmoc deprotection by using twice 1.5 mL of a mixture DMF/piperidine 70/30. Four equivalents of Fmoc-Lys(Fmoc)-OH activated by PyBop and HOBt and eight equivalents of *N,N-*diisopropylethylamine (DIPEA) were added in DMF. After the Fmoc deprotection two residues of Fmoc-Lys(Fmoc)-OH were coupled in the same way on the two lysine amino functions to give the fully protected (Fmoc-Lys(Fmoc))₂Lys-Gly-CCK8-resin. A small amount of this product was treated for deprotection and cleavage and the crude peptide (Lys)₂Lys-Gly-CCK8 was analyzed by Mass spectrum and HPLC. After confirmation of the peptide identity the fully protected (Fmoc-Lys(Fmoc))₂Lys-Gly-CCK8-resin was Fmoc deprotected and DTPA-GLU coupled. The DTPA-GLU coupling was performed by using 2 equivalents of N,N-bis[2-[bis[2-(1,1-dimethylethoxy)-2-oxoethyl]amino]ethyl]-L-glutamic acid 1-(1,1-dimethylethyl) ester (the protected DTPA-GLU), activated by PyBop and HOBt and 4 equivalents of DIPEA in DMF; the stirring time was 8 hours in a single coupling. For deprotection and cleavage, the fully protected conjugate peptide resin was treated with TFA containing triisopropylsilane (2.0%), ethandithiole (2.5%) and water (1.5%). The crude products precipitated at 0°C by adding diethyl ether dropwise. Purifications of the crude mixtures were carried out by RP-HPLC and gave two main products:

(DTPA-GLU)₃(Lys)₂Lys-Gly-CCK8, Rt =23.6 min

(DTPA-GLU)₄(Lys)₂Lys-Gly-CCK8, Rt =24.0 min

Mass spectra confirmed the product identities:

(DTPA-GLU)-(Lys)₂Lys-Gly-CCK8, MW = 2843 (calcd 2842);

(DTPA-GLU)₄(Lys)₂Lys-Gly-CCK8, MW = 3293 (calcd 3292);

### B) Synthesis of the gadolinium complex of compound (DTPA-GLU)₄(Lys)₂Lys-Gly-CCK8.

A 1 mM solution of GdCl₃ was added to the solution of the compound, obtained in step A above, at room temperature, continuously controlling the solution pH with a further addition of a NaOH solution.
The MS was consistent with the indicated structure.

### Example 2

### Synthesis of the gadolinium complex of (DTPA-GLU)₃(Lys)₂Lys-Gly-CCK8

A 1 mM solution of GdCl₃ was added to the solution of (DTPA-GLU)₃(Lys)₂Lys-Gly-CCK8 (prepared as indicated above in Example 1 step A) at room temperature, continuously controlling the solution pH with a further addition of a NaOH solution.
The MS was consistent with the indicated structure.

### Example 3

### Synthesis of the gadolinium complex of (DTPAGLU)₄(Lys)₂Lys-Gly-Vapreotide

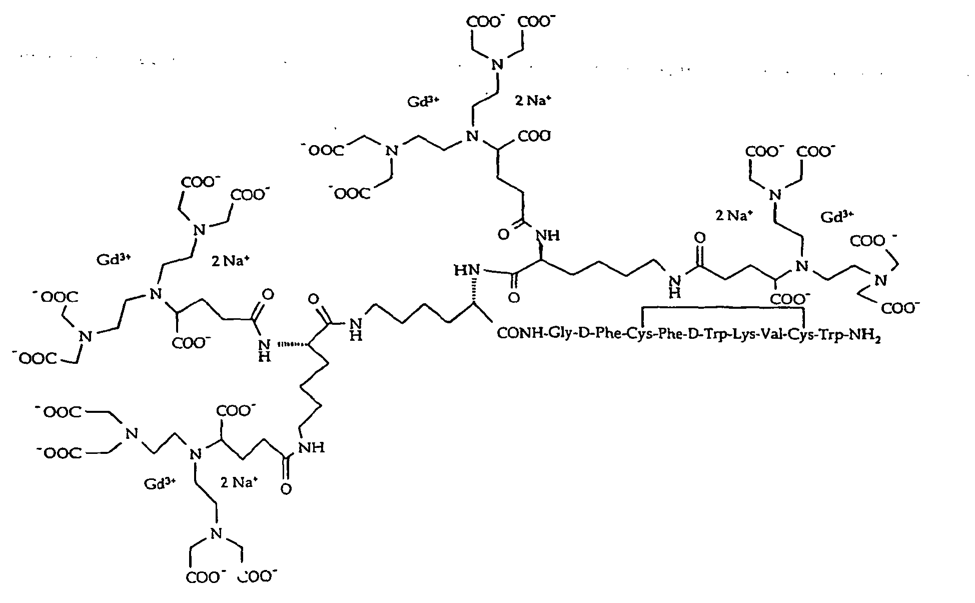

### A) Synthesis of (DTPAGLU)₄(Lys)₂Lys-Gly-Vapreotide

The same procedure described in the Example 1 was followed. (Vapreotide sequence: D-Phe-Cys-Phe-D-Trp-Lys-Val-Cys-Trp-NH₂, Cys² - Cys⁷ S-S bridge). The cyclization of cystein bridge was carried out in 0.5 mM aqueous solution of dimethylsulfoxide (DMSO). Purification of the crude mixture was carried out by RP-HPLC and gave (DTPA-GLU)₄(Lys)₂Lys-Gly-Vapreotide as main product.

(DTPA-GLU)₄(Lys)₂Lys-Gly-Vapreotide Rt= 25.0 min;MW = 3336 (calcd 3335)

The MS was consistent with the indicated structure.

### B) Synthesis of the gadolinium complex of (DTPAGLU)₄(Lys)₂Lys-Gly-Vapreotide

A 1 mM solution of GdCl₃ was added to the solution of the compound of Example 3A at room temperature, continuously controlling the solution pH with a further addition of a NaOH solution.
The MS was consistent with the indicated structure.
The relaxivity of the complex was 15 mM⁻¹ s⁻¹.

### Example 4

### Synthesis of the gadolinium complex of (DO3A-Ar)₄(Lys)₂Lys-Gly-Vapreotide

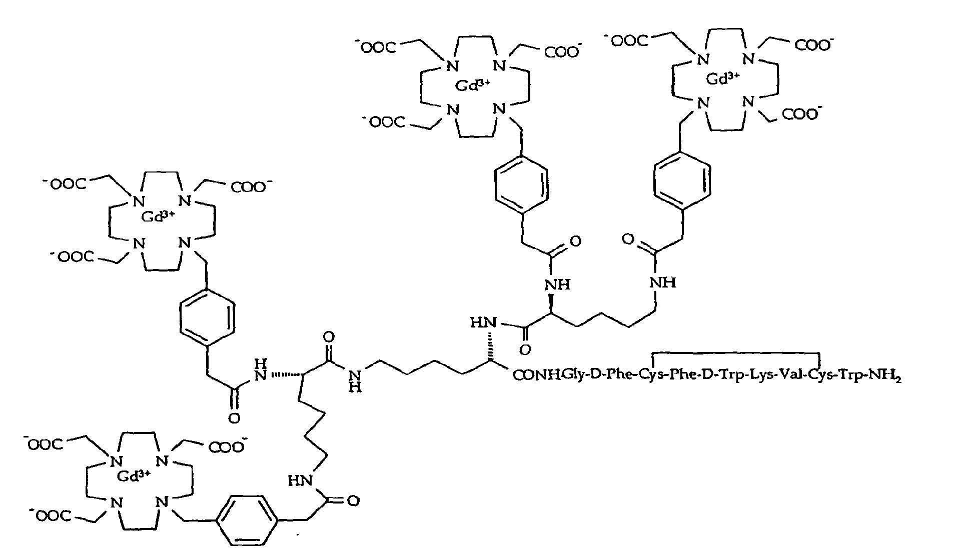

wherein; DO3A-Ar =

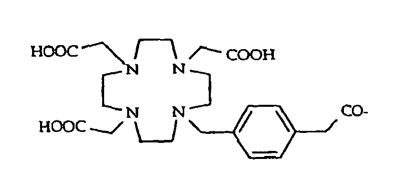

### A) Synthesis of 1,4,7,10-tetraazacyclododecane-1,4,7-triacetic tris(1,1-dimethylethyl) ester

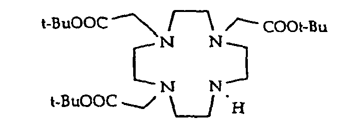

The title product was obtained as disclosed in WO 95/27705. The 10-formyl derivative of 1,4,7 10-tetraazacyclododecane-1,4,7-triacetic tris(1,1-dimethylethyl) ester (Inorg. Chem. 30, 1265-1269, 1991) was deformylated with hydroxylamine hydrochloride in refluxing anhydrous EtOH, to obtain D03A tris(1,1-dimethylethyl) ester monohydrochloride.

This product was dissoved in CH₂Cl₂ and aqueous K₂CO₃. The organic phase was separated, dried on Na₂SO₄, filtered and evaporated to give D03A tris(1,1-dimethylethyl) ester as free base.

### B) Synthesis of 10-[[4-(carboxymethyl)phenyl]methyl]-1,4,7,10-tetraazacyclododecan-1,4,7-triacetic acetic tris(1,1-dimethylethyl) ester

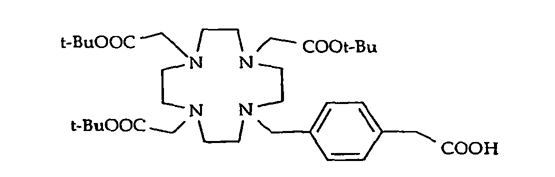

A suspension of D03A tris(1,1-dimethylethyl)ester in 5 mL of ethanol was added to a solution of 4-(bromomethyl)phenylacetic acid (0.934 mmol, 214 mg) in 5.0 mL of ethanol containing 0.234 mL of KOH 4 M. The pH was maintained to 10-11 by the addition of KOH 4 M, and the reaction mixture was warmed for 18 hours at 70°C. After removing the solvent under vacuum, the crude was purified on silica gel by using a mixture of CH₂Cl₂ and methanol (90:10) as the eluent. The product was obtained as a white powder (Yield 80 %). The MS and ¹H-NMR were consistent with the indicated structure.

### C) Synthesis of the gadolinium complex of (DO3A-Ar)₄(Lys)₂Lys-Gly-Vapreotide

The chelating compound and the corresponding gadolinium complex were obtained according to the method described in Example 3.
The MS was consistent with the indicated structure.

### Example 5

### Synthesis of the gadolinium complex of (Lys(DTPA-GLU)-βAla)₄ Lys(DTPA-GLU)-Gly-CCK8

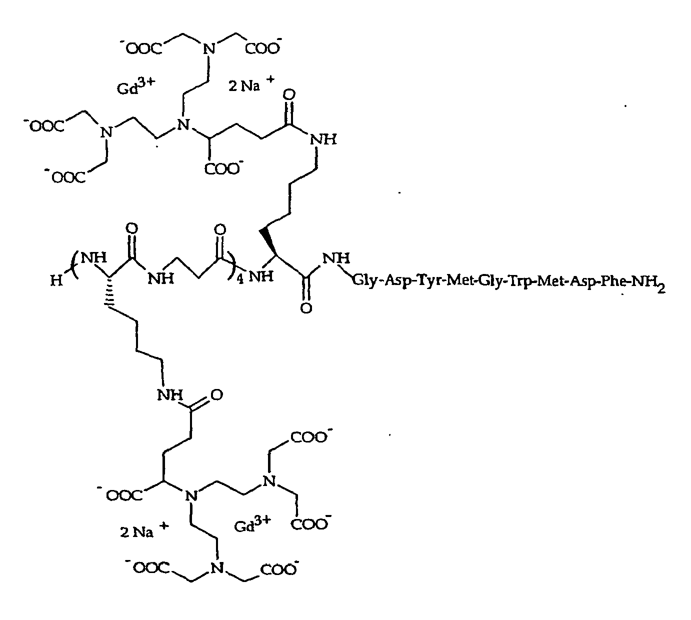

### A) Synthesis of (Lys(DTPA-GLU)- βAla)₄ Lys(DTPA-GLU)-Gly-CCK8

Gly-CCK8 (sequence: H-Gly-Asp-Tyr-Met-Gly-Trp-Met-Asp-Phe-NH₂.) The synthesis was carried out in solid phase under standard conditions using the Fmoc protocol. The Rink-amide MBHA resin (0.54 mmol/g, 54 µmol scale, 0.100 g) was used. Double couplings were performed, adding each time four equivalents of N-protected amino acid activated by PyBop and HOBt and eight equivalents of *N,N*-diisopropylethylamine (DIPEA) in DMF, and stirring for 60 min. When the peptide GlyCCK8 synthesis was complete, the resin was transferred in a vessel for Fmoc deprotection and four equivalents of Fmoc-Lys(Mtt)-OH activated by PyBop and HOBt and eight equivalents of *N,N-*diisopropylethylamine (DIPEA) were added in DMF. The Mtt Lys side-chain protecting group was removed by treatment with a TFA/triisopropylsilane/CH₂Cl₂ (1:5:94) mixture; then N,N-bis[2-[bis[2-(1,1-dimethylethoxy)-2-oxoethyl]amino]ethyl]-L-glutamic acid 1-(1,1-dimethylethyl) ester (the protected DTPA-GLU), activated by HATU and 4 equivalents of DIPEA in DMF was coupled to the Lys ε-amino group. The Fmoc protecting group was removed and four equivalents of FmocβAla-OH activated by PyBop and HOBt and eight equivalents of *N,N-*diisopropylethylamine (DIPEA) in DMF were added. The same procedure beginning from the Fmoc-Lys(Mtt)-OH was repeated respectively four times to achieve the fully protected product (Lys(DTPA-GLU)-βAla)₄-Lys(DTPA-GLU)-Gly-CCK8 [or nine times to achieve the fully protected product (Lys(DTPA-GLU)-βAla)₉-Lys(DTPA-GLU)-Gly-CCK8, see example 6]. For deprotection and cleavage, the fully protected conjugate peptide resins were treated with TFA containing triisopropylsilane (2.0%), ethandithiole (2.5%) and water (1.5%). The crude products were precipitated at 0°C by adding diethyl ether dropwise. Purifications of the crude mixtures were carried out by RP-HPLC using the reported methods. Compound (Lys(DTPA-GLU)-βAla)₄-Lys(DTPA-GLU)-Gly-CCK8 was obtained in good yield (40% for the HPLC purified compound) and high purity (95% by HPLC).

(Lys(DTPA-GLU)-βAla)₄ -Lys(DTPA-GLU)-Gly-CCK8 Rt =23.5 min

The MS is consistent with the indicated structure.

### B) Synthesis of the gadolinium complex of (Lys(DTPA-GLU)-βAla)₄ Lys(DTPA-GLU)-Gly-CCK8

The preparation of the gadolinium complex was obtained according to the method described in Example 1.
The MS was consistent with the indicated structure.

### Example 6

### Synthesis of the gadolinium complex of (Lys(DTPA-GLU)-βAla)₉ Lys(DTPA-GLU)-Gly-CCK8

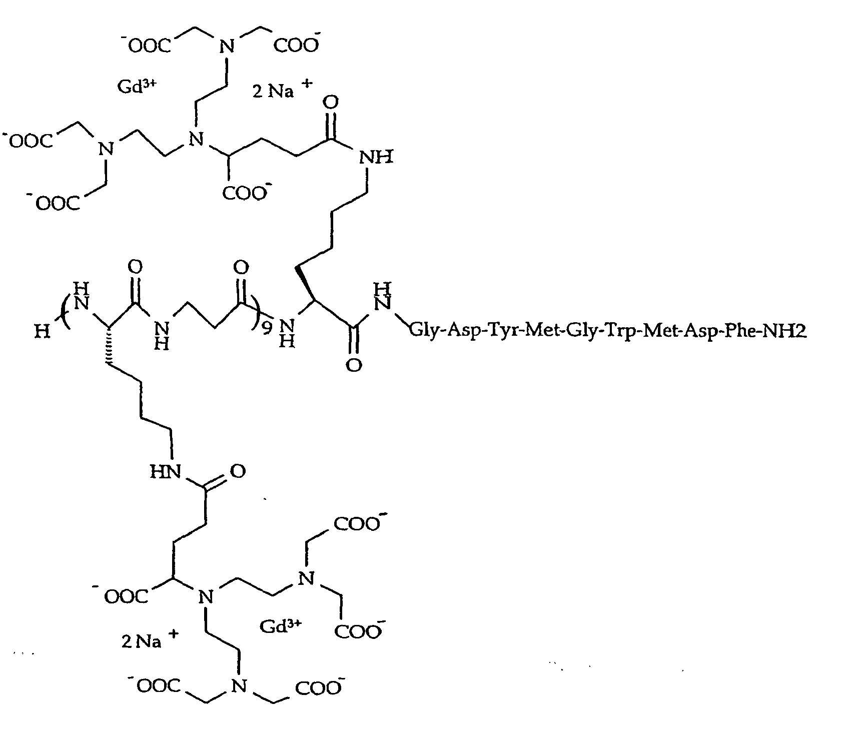

(Lys(DTPA-GLU)-βAla)₉-Lys(DTPA-GLU)-Gly-CCK8 was prepared according to the procedure given in detail in the Example 5, in 20 % yield.
(Lys(DTPA-GLU)-βAla)₉-Lys(DTPA-GLU)-Gly-CCK8 Rt =32.0 min.
The MS is consistent with the indicated structure.

### Example 7

### Synthesis of the gadolinium complex of (Lys(DTPA-GLU)-βAla)₄ Lys(DTPA-GLU)-Gly-Vapreotide

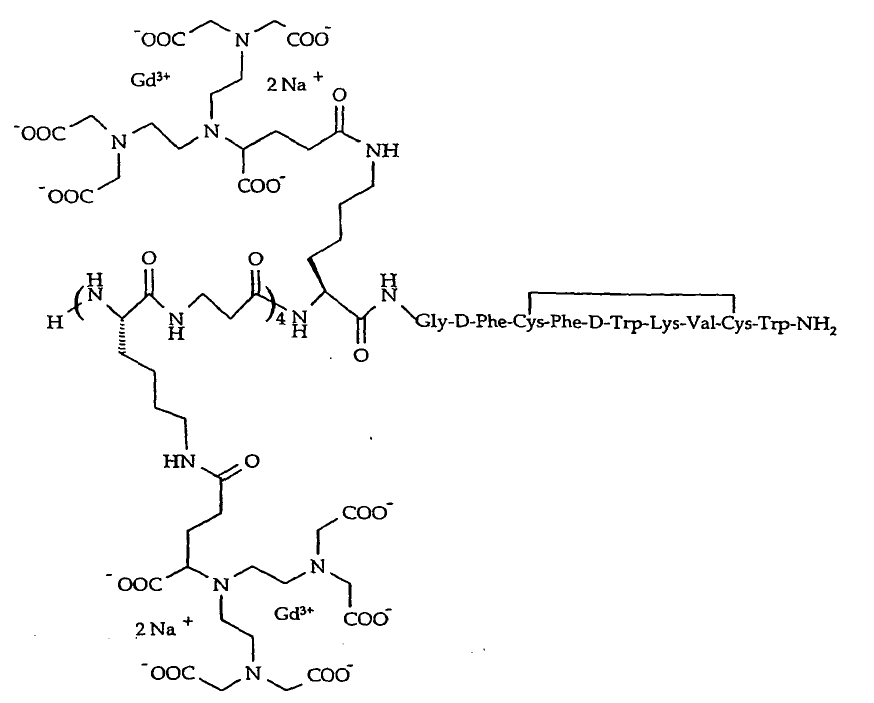

The chelating compound and the corresponding gadolinium complex are prepared according to the procedure given in detail in Example 5.
The MS was consistent with the indicated structure.

### Example 8

### Synthesis of the gadolinium complex of (Lys(DTPA-GLU)-βAla)₉ Lys(DTPA-GLU)-Gly-Vapreotide

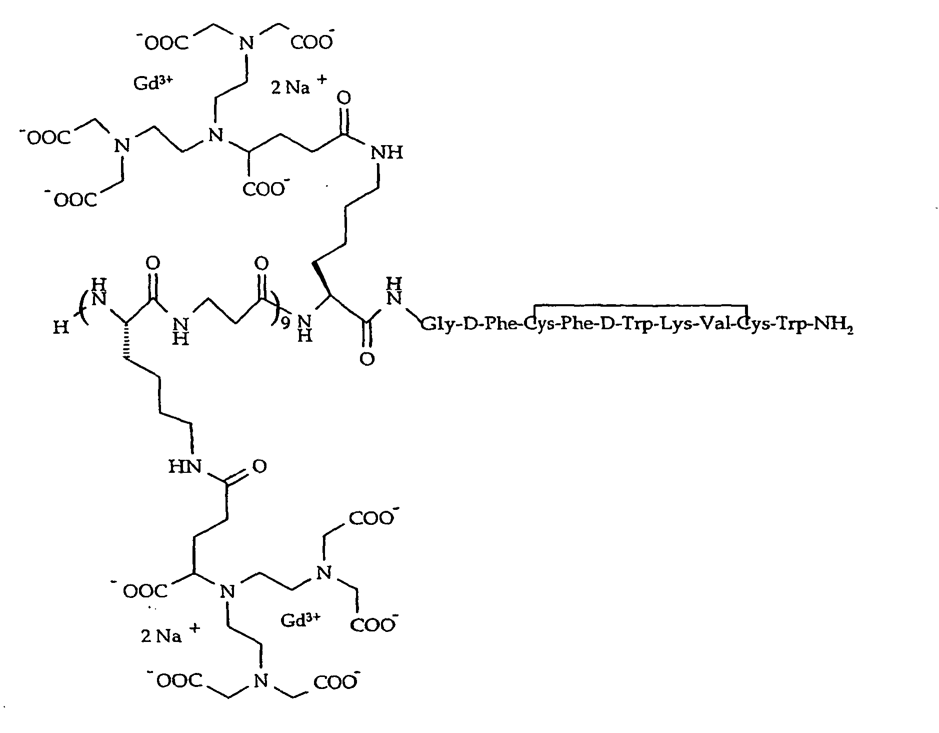

The chelating compound and the corresponding gadolinium complex were prepared according to the procedure given in the Example 5.
The MS was consistent with the indicated structure.

### Example 9

### Synthesis of the gadolinium complex of (Dap(DTPA-GLU)-βAla)₄₋Dap(DTPA-GLU)-Gly-CCK8

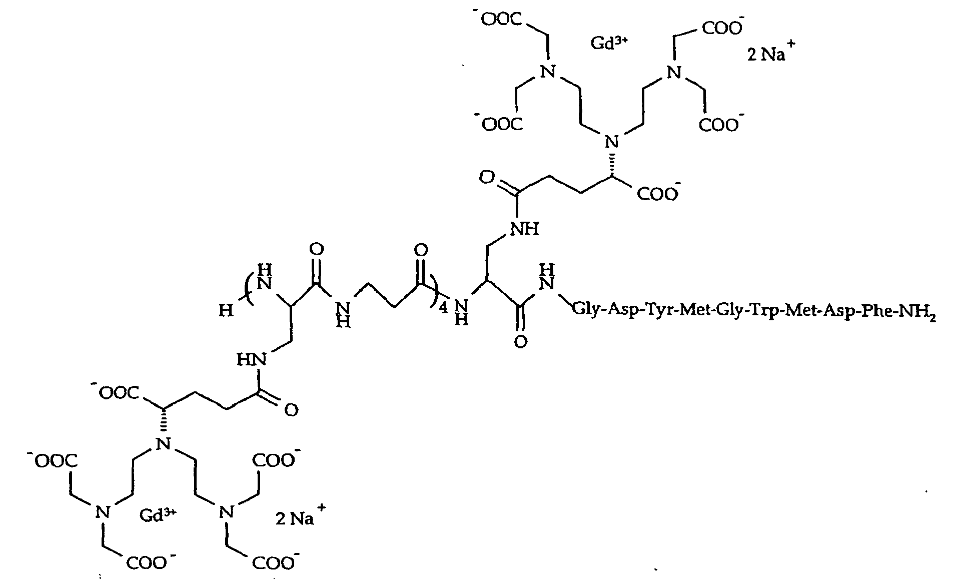

### A) Preparation of 3-[[(4S)-4-[bis[2-[bis[2-(1,1-dimethylethoxy)-2-oxoethyl]amino]ethyl]amino]-5-(1,1-dimethylethoxy)-1,5-dioxopentyl]amino]-N-[(9H-fluoren-9-ylmethoxy)carbonyl]-L-alanine

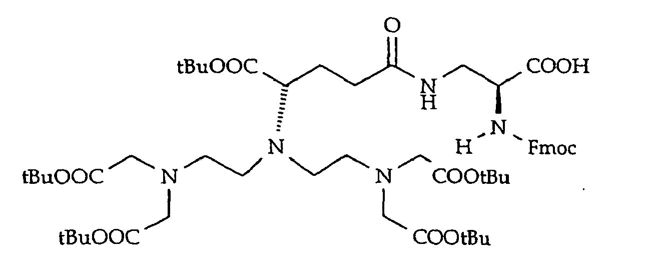

A solution of *N*-(3-dimethylaminopropyl)-*N'*-ethylcarbodiimide hydrochloride (EDCI) (2.30 g; 12 mmol) in CH₂Cl₂ (40 mL) was added to a solution of *N,N-*bis[2-[bis[2-(1,1-dimethylethoxy)-2-oxoethyl]amino]ethyl]-L-glutamic acid 1-(1,1-dimethylethyl) ester (7.46 g; 10 mmol) and N-hydroxysuccinimide (NHS) (1.38 g; 12 mmol) stirred at 0-5°C. After 18 h at room temperature the solution was washed with H₂O, dried (Na₂SO₄) and evaporated to give the intermediate activated ester. The latter was dissolved in DMF (100 mL) and 1.01 g of triethylamine was added then a solution of 3-amino-*N*-[(9*H*-fluoren-9-ylmethoxy)carbonyl]-L-alanine (Fmoc-L-Dap) (3.26 g; 10 mmol) in DMF (100 mL) was added dropwise over 40 min maintaining the reaction mixture at 10-15°C. After 18 h at room temperature, the solution was diluted with CH₂Cl₂ (300 mL) and washed with a solution of 0.1M HCl and H₂O and then with H₂O.

After drying, the solution was evaporated to give a crude that was purified by flash-chromatography to afford the title compound (7.76 g; 7.4 mmol) as a whitish solid in 74% yield.
The IR, ¹H-NMR and MS were consistent with the indicated structure.

### B) Synthesis of (Dap(DTPA-GLU)-βAla)₄-Dap(DTPA-GLU)-Gly-CCK8

The compound was prepared following the strategy described in Example 5 using the Fmoc protocol and alternatively compound prepared in A) of the present example and Fmoc-βAla.
The MS was consistent with the indicated structure.

### C) Synthesis of the gadolinium complex of (Dap(DTPA-GLU)-βAla)₄₋Dap(DTPA)-Gly-CCK8.

The complex was prepared according to the procedure given in detail in the Example 1.
The MS was consistent with the indicated structure.

### Example 10

### Synthesis of the gadolinium complex of (Dap(DTPA-GLU)-βAla)₉₋Dap(DTPA-GLU)-Gly- CCK8

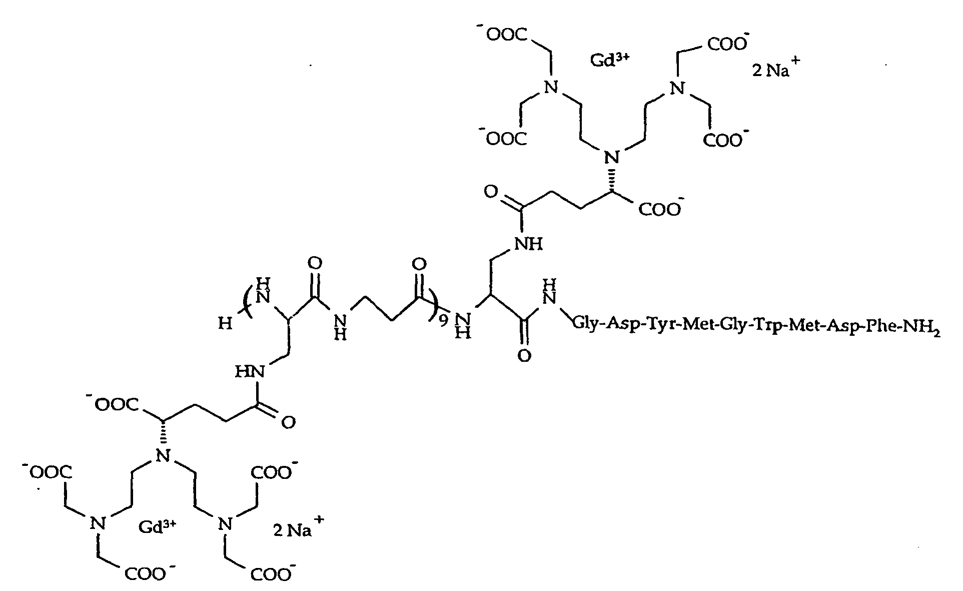

The title compound was prepared according to the procedure used in the Example 9.
The MS was consistent with the indicated structure.

### Example 11

### Synthesis of the gadolinium complex of (Dap(DTPA-GLU)-βAla)₄₋Dap(DTPA-GLU)-Vapreotide

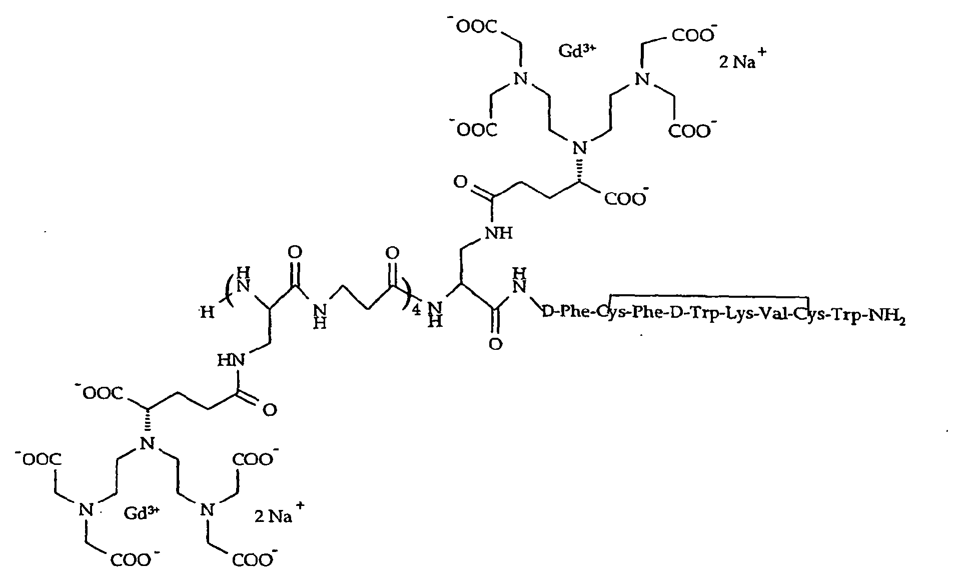

The title compound was prepared according to the procedure given in the Example 9. The cyclization of cystein bridge was carried out according to the methodology indicated in the Example 3.
The MS was consistent with the indicated structure.

### Example 12

### Synthesis of the gadolinium complex of (Dap(DTPA-GLU)-βAla)₉₋Dap(DTPA-GLU)-Vapreotide

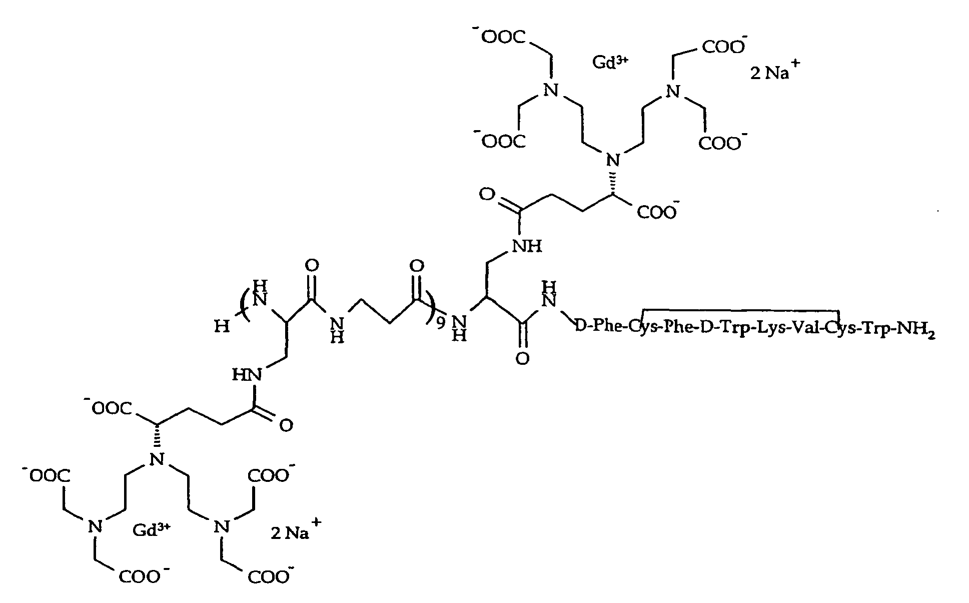

The title compound was prepared according to the procedure given in the Examples 9 and 11.
The MS was consistent with the indicated structure.

### Example 13

### Synthesis of the gadolinium complex of formula

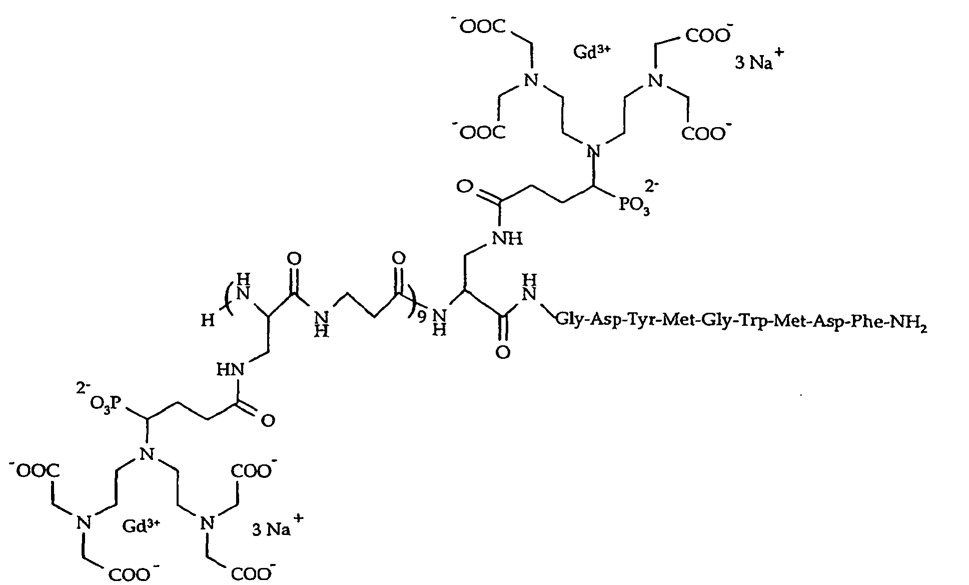

The compound was prepared according to the procedure given in Example 5 using the following protected chelating compound: 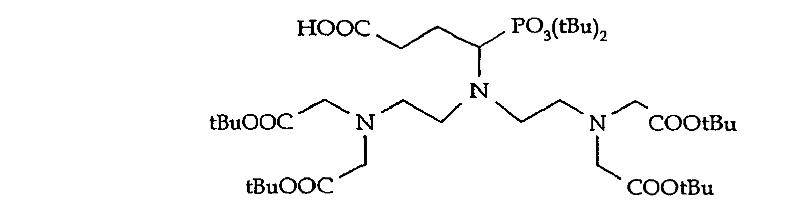 The MS was consistent with the indicated structure.

### Example 14

### Synthesis of the gadolinium complex of formula

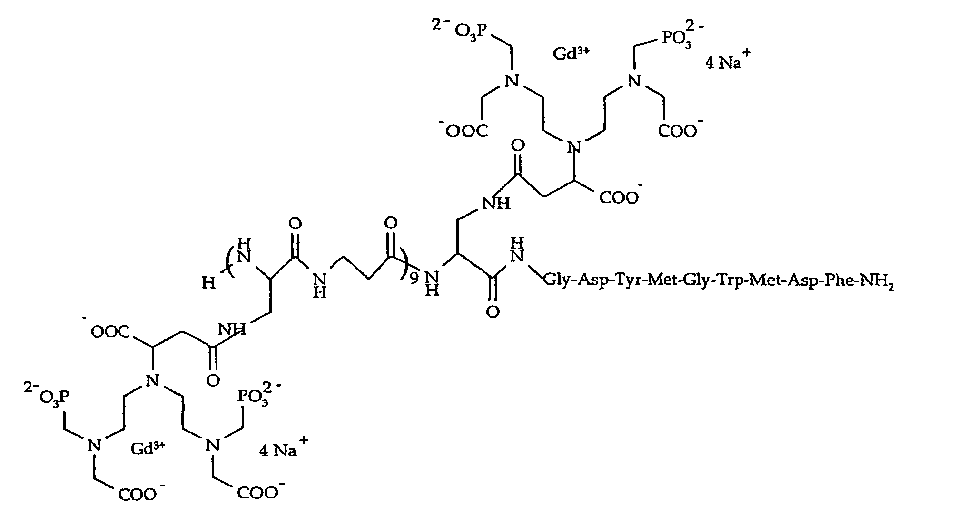

The compound was prepared according to the procedure given in Example 5 using the following protected chelating compound: 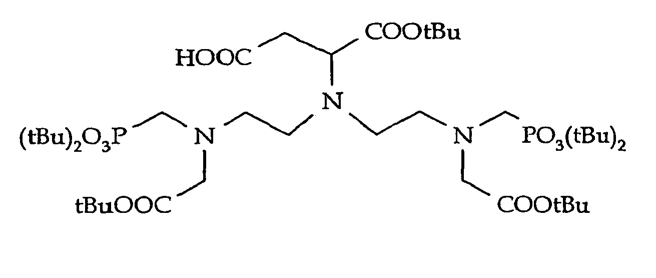 The MS was consistent with the indicated structure.

## Claims

1. Compounds of general formula (I):
[A]ₜ[P]-[B]ᵥ (I)
wherein:
A is selected from the group consisting of:
ethylenediaminotetraacetic acid (EDTA), diethylenetriaminopentaacetic acid (DTPA), 1,4,7,10-tetraazacyclododecane-1,4,7,10-tetraacetic acid (DOTA), 1,4,7,10-tetraazacyclododecane-1,4,7-triacetic acid (DO3A), [10-(2-hydroxypropyl)-1,4,7,10-tetraazacyclododecane-1,4,7-triacetic acid (HPDO3A), 4-carboxy-5,8,11-tris(carboxymethyl)-1-phenyl-2-oxa-5,8,11-triazatridecan-13-oic acid (BOPTA), N-[2-[bis(carboxymethyl)amino]-3-(4-ethoxyphenyl)propyl]-N-[2-[bis(carboxymethyl)amino]ethylglycine (EOB-DTPA), N,N-bis[2-(carboxymethyl)[(methylcarbamoyl)methyl]amino]ethyl]-glycine (DTPA-BMA), 2-methyl-1,4,7,10-tetraazacyclododecane-1,4,7,10-tetraacetic acid (MCTA), (α,α',α",α"')-tetramethyl-1,4,7,10-tetraazacyclododecane-1,4,7,10-tetracetic acid (DOTMA); the residue of a polyaminophosphonic acid ligand and derivatives thereof, polyaminophosphinic acid and derivatives thereof, in particular ethylenedinitrilotetrakis(methylphosphonic) acid (EDTP); 1,4,7,10-tetraazacyclododecane-1,4,7,10-tetrakis[methylene(methylphosphonic)] acid and 1,4,7,10-tetraazacyclododecane-1,4,7,10-tetrakis[methylene-(methylphosphinic)] acid.
t is an integer ranging from 3 to 15;
B is a peptide selected from:
Gly-Glu-Tyr-Met-Gly-Trp-Met-Asp-Phe-NH₂
Gly-Asp-Tyr-Met-Gly-Trp-Leu-Asp-Phe-OH
Gly-Glu-Tyr(SO₃H)-Met-Gly-Trp-Leu-Asp-Phe-NH₂ Gly-Glu-Cys-Ala-D-Trp-Lys-Leu-Cys-Phe-OH Gly-Ala-Cys-Gly-D-Trp-Lys-Leu-Cys-Phe-NH2 Gly-Ile-Cys-Ala-D-Trp-Lys-Glu-Cys-Lys-NH2
v is an integer of 1 to 5;
P is a polylysine or a poly-L-lysine or a Lys-β-Ala or Dap-β-Ala copolymer.

2. Compounds as claimed in claim 1 in the form of complexes with the bi- trivalent ions of metal elements having atomic numbers ranging from 21 to 29, 42, 44 or from 57 to 71, with paramagnetic metals, in particular with Fe, Cu, Cr, Eu, Gd, Tb, Dy, Ho, Er, Tm, Yb, Mn, as well as the salts thereof with physiologically compatible bases or acids.

3. Compounds as claimed in claim 2 in the form of complexes with the bi- trivalent ions of Fe(2+), Fe(3+), Cu(2+), Cr(2+), Cr(3+), Eu (3+), Gd(3+), Tb(3+), Dy(3+), Ho(3+), Er(3+), Yb(3+), Mn(2+) and Mn(3+) as well as the salts thereof with physiologically compatible bases or acids.

4. Compounds as claimed in claim 3 in the form of complexes obtained with the bi-trivalent ions of Eu(3+), Gd(3+), Dy(3+), Mn(2+) and Mn(3+), as well as the salts thereof with physiologically compatible bases or acids.

5. Compounds as claimed in any one of claims 1 to 4, wherein A is selected from:

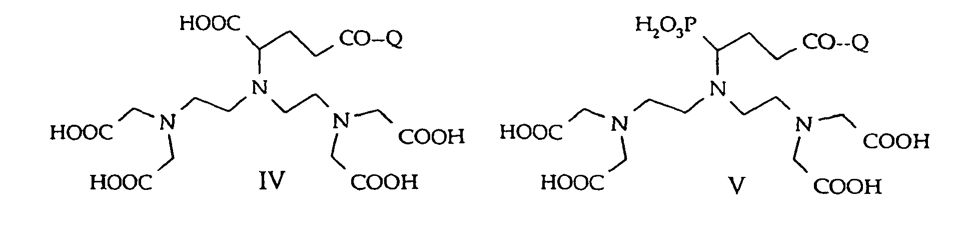

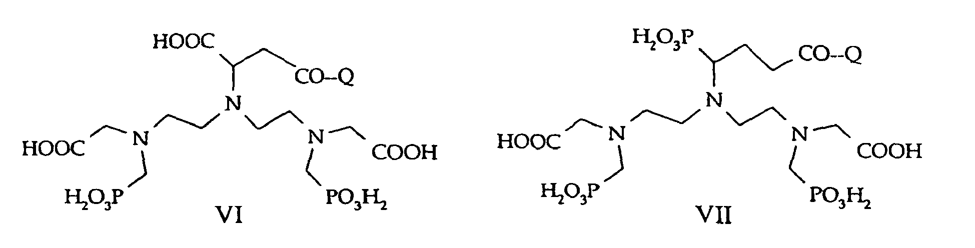

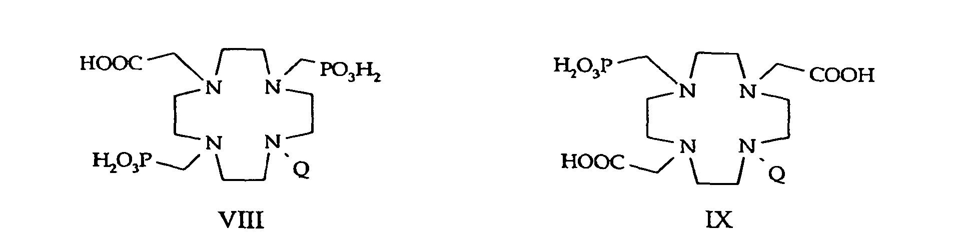

6. Compounds as claimed in any one of the above claims, which are selected from:
(DTPA-GLU)₄(Lys)₂Lys-Gly-CCK8
(DTPA-GLU)₃(Lys)₂Lys-Gly-CCK8
(DTPA-GLU)₄(Lys)₂Lys-Gly-Vapreotide
(DO3A-Ar)₄(Lys)₂Lys-Gly-Vapreotide
(Lys(DTPA-GLU)- βAla)₄ Lys(DTPA-GLU)-Gly-CCK8
(Lys(DTPA-GLU)-βAla)₉ Lys(DTPA-GLU)-Gly-CCK8
(Lys(DTPA-GLU)-βAla)₄ Lys(DTPA-GLU)-Gly-Vapreotide
(Lys(DTPA-GLU)-βAla)₉ Lys(DTPA-GLU)-Gly-Vapreotide
(Dap(DTPA-GLU)-βAla)₄-Dap(DTPA-GLU)-Gly-CCK8
(Dap(DTPA-GLU)-βAla)₉-Dap(DTPA-GLU)-Gly-CCK8
(Dap(DTPA-GLU)-βAla)₄-Dap(DTPA-GLU)-Vapreotide
(Dap(DTPA-GLU)-βAla)₉-Dap(DTPA-GLU)-Vapreotide

7. Pharmaceutical and diagnostic composition containing a compound of claims 1-6 in admixture with a suitable carrier.

8. The use of the compounds of claims 1 to 6 and of the salts thereof for the preparation of diagnostic formulations used in MRI investigations, for imaging and recording images of organs and/or tissues.

9. The use as claimed in claim 8, for *in vitro* and/or *in vivo* imaging and recording images of cells, tissues or organs in which tumours or primary tumour pathologies or metastases are present.

10. Compounds of formula:
A'-Dap-Fmoc
wherein A' is a unit of formula A as defined above having the carboxy or phosphonic moieties suitably protected, Dap is a diamino-acid, specifically 2,3-diaminopropionic acid and Fmoc is (9H-fluoren-9-ylmethoxy)carbonyl

11. A compound according to claim 10, which is 3-[[(4*S*)-4-[bis[2-[bis[2-(1,1-dimethylethoxy)-2-oxoethyl]amino]ethyl]amino]-5-(1,1-dimethylethoxy)-1,5-dioxopentyl]amino]-*N*-[(9*H*-fluoren-9-ylmethoxy)carbonyl]-L-alanine.

## Patentansprüche

1. Verbindungen der allgemeinen Formel (I):
[A] ₜ [P] - [B] ᵥ (I)
worin:
A aus der Gruppe, bestehend aus:
Ethylendiaminotetraessigsäure (EDTA), Diethylentriaminopentaessigsäure (DTPA), 1,4,7,10-Tetraazacyclododecan-1,4,7,10-tetraessigsäure (DOTA), 1,4,7,10-Tetraazacyclododecan-1,4,7-triessigsäure (D03A), [10-(2-Hydroxypropyl)-1,4,7,10-tetraazacyclododecan-1,4,7-triessigsäure (HPD03A), 4-Carboxy-5,8,11-tris(carboxymethyl)-1-phenyl-2-oxa-5,8,11-triazatridecan-13-onsäure (BOPTA), N-[2-[Bis(carboxymethyl)amino]-3-(4-ethoxyphenyl)propyl]-N-[2-[bis(carboxymethyl)amino]ethylglycin (EOB-DTPA), N,N-Bis[2-(carboxymethyl)[(methylcarbamoyl)methyl]amino]-ethyl]glycin (DTPA-BMA), 2-Methyl-1,4,7,10-tetraazacyclododecan-1,4,7,10-tetraessigsäure (MCTA), (α,α',α'',α'''.)-Tetramethyl-1,4,7,10-tetraazacyclododecan-1,4,7,10-tetraessigsäure (DOTMA); dem Rest eines Polyaminophosphonsäureliganden und Derivaten davon, Polyaminophosphinsäure und Derivaten davon, insbesondere Ethylendinitrilotetrakis(methylphosphon)säure (EDTP); 1,4,7,10-Tetraazacyclododecan-1,4,7,10-tetrakis[methylen(methylphosphon)]säure und 1,4,7,10-Tetraazacyclododecan-1,4,7,10-tetrakis[methylen(methylphosphin)]säure, ausgewählt ist;
t eine ganze Zahl im Bereich von 3 bis 15 ist;
B ein Peptid, ausgewählt aus:
Gly-Glu-Tyr-Met-Gly-Trp-Met-Asp-Phe-NH₂
Gly-Asp-Tyr-Met-Gly-Trp-Leu-Asp-Phe-OH
Gly-Glu-Tyr(SO₃H)-Met-Gly-Trp-Leu-Asp-Phe-NH₂ 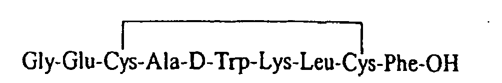
Gly-Glu-Cys-Ala-D-Trp-Lys-Leu-Cys-Phe-OH
 Gly-Ala-Cys-Gly-D-Trp-Lys-Leu-Cys-Phe-NH2 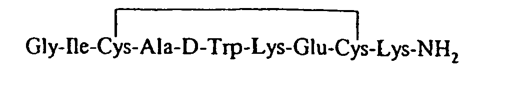
Gly-Ile-Cys-Ala-D-Trp-Lys-Glu-Cys-Lys-NH2
ist,
v eine ganze Zahl von 1 bis 5 ist;
P ein Polylysin oder ein Poly-L-lysin oder ein Lys-β-Ala- oder ein Dap-β-Ala-Copolymeres ist.

2. Verbindungen nach Anspruch 1 in der Form von Komplexen mit zwei-/dreiwertigen Ionen von Metallelementen mit Atomzahlen im Bereich von 21 bis 29, 42, 44 oder von 57 bis 71, mit paramagnetischen Metallen, insbesondere mit Fe, Cu, Cr, Eu, Gd, Tb, Dy, Ho, Er, Tm, Yb, Mn, sowie die Salze davon mit physiologisch verträglichen Basen oder Säuren.

3. Verbindungen nach Anspruch 2 in Form von Komplexen mit den zwei-/dreiwertigen Ionen von Fe(2+), Fe(3+), Cu(2+), Cr(2+), Cr(3+), Eu(3+), Gd(3+), Tb(3+), Dy(3+), Ho(3+), Er(3+), Yb(3+), Mn(2+) und Mn(3+) sowie die Salze davon mit physiologisch verträglichen Basen oder Säuren.

4. Verbindungen nach Anspruch 3 in Form von Komplexen, erhalten mit zwei-/dreiwertigen Ionen von Eu(3+), Gd(3+), Dy(3+), Mn(2+) und Mn(3+) sowie die Salze davon mit physiologisch verträglichen Basen oder Säuren.

5. Verbindungen nach einem der Ansprüche 1 bis 4, wobei A aus: 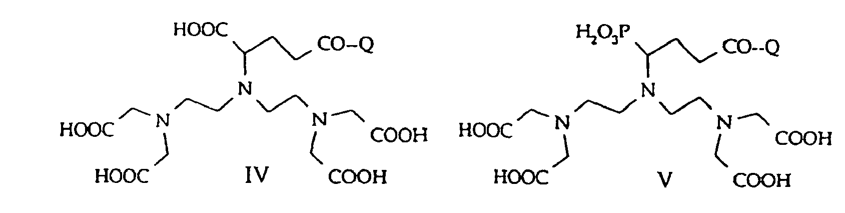 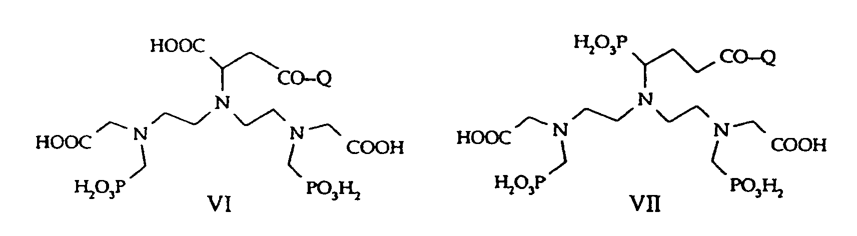 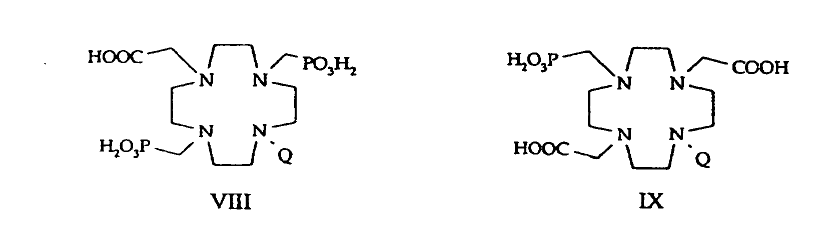 ausgewählt ist.

6. Verbindungen nach einem der obigen Ansprüche, nämlich solche, ausgewählt aus:
(DTPA-GLU)₄(Lys)₂Lys-Gly-CCK8
(DTPA-GLU)₃(Lys)₂Lys-Gly-CCK8
(DTPA-GLU)₄(Lys)₂Lys-Gly-Vapreotid
(DO3A-Ar)₄(Lys)₂Lys-Gly-Vapreotid
(Lys(DTPA-GLU)-βAla)₄ Lys(DTPA-GLU)-Gly-CCK8
(Lys(DTPA-GLU)-βAla)₉ Lys(DTPA-GLU)-Gly-CCK8
(Lys(DTPA-GLU)-βAla)₄ Lys(DTPA-GLU)-Gly-Vapreotid
(Lys(DTPA-GLU)-βAla)₉ Lys(DTPA-GLU)-Gly-Vapreotid
(Dap(DTPA-GLU)-βAla)₄-Dap(DTPA-GLU)-Gly-CCK8
(Dap(DTPA-GLU)-βAla)₉-Dap(DTPA-GLU)-Gly-CCK8
(Dap(DTPA-GLU)-βAla)₄-Dap(DTPA-GLU)-Vapreotid
(Dap(DTPA-GLU)-βAla)₉-Dap(DTPA-GLU)-Vapreotid

7. Pharmazeutische und diagnostische Zusammensetzung, enthaltend eine Verbindung nach den Ansprüchen 1 bis 6 im Gemisch mit einem geeigneten Träger.

8. Verwendung der Verbindungen nach den Ansprüchen 1 bis 6 und der Salze davon zur Herstellung von diagnostischen Zubereitungen, die für MRI-Untersuchungen zur Abbildung und Aufzeichnung von Bildern von Organen und/oder Geweben verwendet werden.

9. Verwendung nach Anspruch 8 für das *in vitro* und/oder *in vivo* erfolgende Abbilden und Aufzeichnen von Bildern von Zellen, Geweben oder Organen, in denen Tumore oder primäre pathologische Tumorzustände oder Metastasen vorhanden sind.

10. Verbindungen der Formel:
A'-Dap-Fmoc
worin A' eine Einheit der oben angegebenen Formel A, bei der die Carboxy- oder Phosphongruppierungen in geeigneter Weise geschützt sind, bedeutet, Dap für eine Diaminosäure, speziell 2,3-Diaminopropionsäure, steht und Fmoc für (9H-Fluoren-9-ylmethoxy)carbonyl steht.

11. Verbindung nach Anspruch 10, nämlich 3-[((4*S*)-4-[Bis[2-[bis[2-(1,1-Dimethylethoxy)-2-oxoethyl]amino]-ethyl]amino]-5-(1,1-dimethylethoxy)-1,5-dioxopentyl]-amino]-N-[(9H-fluoren-9-ylmethoxy)carbonyl]-L-alanin.

## Revendications

1. Composés de formule générale (I) :
[A]ₜ[P]-[B]ᵥ (I)
dans laquelle :
A est choisi dans le groupe constitué par :
l'acide éthylènediaminotétraacétique (EDTA), l'acide diéthylènetriaminopentaacétique (DTPA), l'acide 1,4,7,10-tétraazacyclododécane-1,4,7,10-tétraacétique (DOTA), l'acide 1,4,7,10-tétraazacyclododécane-1,4,7-triacétique (D03A), l'acide 10-(2-hydroxypropyl)-1,4,7,10-tétraazacyclododécane-1,4,7-triacétique (HPDO3A), l'acide 4-carboxy-5,8,11-tris(carboxyméthyl)-1-phényl-2-oxa-5,8,11-triazatridécan-13-oïque (BOPTA), la N-[2-[bis(carboxyméthyl)amino]-3-(4-éthoxyphényl)propyl]-N-[2-[bis(carboxyméthyl)amino]éthylglycine (EOB-DTPA), la N,N-bis[2-(carboxyméthyl)[(méthylcarbamoyl)méthyl]amino]éthyl]-glycine (DTPA-BMA), l'acide 2-méthyl-1,4,7,10-tétraazacyclodo-décane-1,4,7,10-tétraacétique (MCTA), l'acide (α,α',α",α"')-tétraméthyl-1,4,7,10-tétraazacyclododécane-1,4,7,10-tétraacétique (DOTMA) ; le résidu d'un ligand poly(acide aminophosphonique) et des dérivés de celui-ci, un poly(acide aminophosphinique) et les dérivés de celui-ci, en particulier l'acide éthylènedinitrilotétrakis(méthyl-phosphonique) (EDTP) ; l'acide 1,4,7,10-tétraazacyclo-dodécane-1,4,7,10-tétrakis[méthylène(méthylphosphonique)] et l'acide 1,4,7,10-tétraazacyclododécane-1,4,7,10-tétrakis[méthylène(méthylphosphinique)].
t est un entier de 3 à 15 ;
B est un peptide choisi parmi :
Gly-Glu-Tyr-Met-Gly-Trp-Met-Asp-Phe-NH₂
Gly-Asp-Tyr-Met-Gly-Trp-Leu-Asp-Phe-OH
Gly-Glu-Tyr(SO₃H)-Met-Gly-Trp-Leu-Asp-Phe-NH₂ Gly-Glu-Cys-Ala-D-Trp-Lys-Leu-Cys-Phe-OH Gly-Ala-Cys-Gly-D-Trp-Lys-Leu-Cys-Phe-NH2 Gly-Ile-Cys-Ala-D-Trp-Lys-Glu-Cys-Lys-NH2
v est un entier de 1 à 5 ;
P est une polylysine ou une poly-L-lysine ou un copolymère Lys-β-Ala ou Dap-β-Ala.

2. Composés tels que revendiqués dans la revendication 1 sous la forme de complexes avec les ions bi-, trivalents d'éléments métalliques ayant des nombres atomiques de 21 à 29, 42, 44, ou de 57 à 71, avec des métaux paramagnétiques, en particulier avec le Fe, le Cu, le Cr, l'Eu, le Gd, le Tb, le Dy, l'Ho, l'Er, le Tm, l'Yb, le Mn, ainsi que les sels de ceux-ci avec des bases ou des acides physiologiquement compatibles.

3. Composés tels que revendiqués dans la revendication 2 sous la forme de complexes avec les ions bi-, trivalents Fe(2+), Fe (3+) , Cu(2+), Cr(2+), Cr(3+), Eu(3+), Gd(3+), Tb(3+), Dy(3+), Ho(3+), Er(3+), Yb(3+), Mn(2+) et Mn(3+), ainsi que les sels de ceux-ci avec des bases ou des acides physiologiquement compatibles.

4. Composés tels que revendiqués dans la revendication 3 sous la forme de complexes obtenus avec les ions bi-, trivalents Eu(3+), Gd(3+), Dy(3+), Mn(2+) et Mn(3+), ainsi que les sels de ceux-ci avec des bases ou des acides physiologiquement compatibles.

5. Composés tels que revendiqués dans l'une quelconque des revendications 1 à 4, pour lesquels A est choisi parmi :

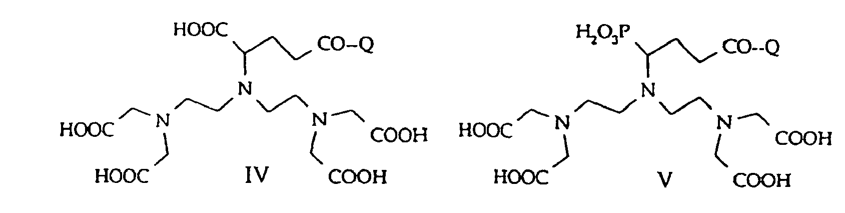

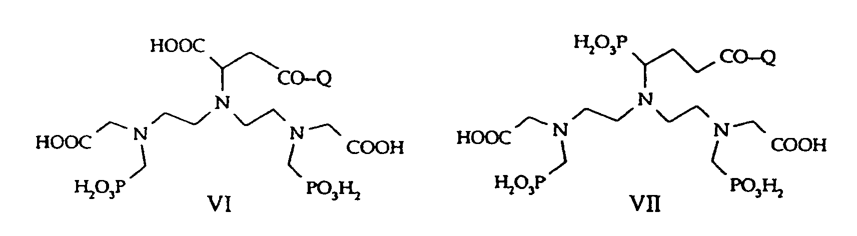

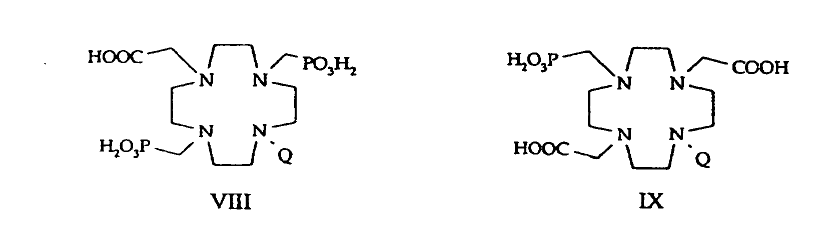

6. Composés tels que revendiqués dans l'une quelconque des revendications ci-dessus, qui sont choisis parmi :
(DTPA-GLU)₄(Lys)₂Lys-Gly-CCK8
(DTPA-GLU)₃(Lys)₂Lys-Gly-CCK8
(DTPA-GLU)₄(Lys)₂Lys-Gly-Vapréotide
(D03A-Ar)₄(Lys)₂Lys-Gly-Vapréotide
(Lys(DTPA-GLU)-βAla)₄Lys(DTPA-GLU)-Gly-CCK8
(Lys (DTPA-GLU) -βAla)₉Lys (DTPA-GLU) -Gly-CCK8
(Lys (DTPA-GLU) -βAla)₄Lys (DTPA-GLU) -Gly-Vapréotide
(Lys (DTPA-GLU) -βAla)₉Lys (DTPA-GLU) -Gly-Vapréotide
(Dap (DTPA-GLU) -βAla)₄-Dap (DTPA-GLU) -Gly-CCK8
(Dap (DTPA-GLU) -βAla)₉-Dap (DTPA-GLU) -Gly-CCK8
(Dap(DTPA-GLU)-βAla)₄-Dap(DTPA-GLU)-Vapréotide
(Dap (DTPA-GLU) -βAla)₉-Dap (DTPA-GLU) -Vapréotide.

7. Composition pharmaceutique et de diagnostic contenant un composés des revendications 1-6 en mélange avec un véhicule approprié.

8. Utilisation des composés des revendications 1 à 6 et des sels de ceux-ci pour la préparation de formulations de diagnostic utilisées dans les investigations par IRM, pour l'imagerie et l'enregistrement d'images d'organes et/ou de tissus.

9. Utilisation telle que revendiquée dans la revendication 8, pour faire l'imagerie et l'enregistrement *in vitro* et/ou *in vivo* d'images de cellules, de tissus ou d'organes dans lesquels des tumeurs ou des pathologies de tumeurs primaires ou des métastases sont présentes.

10. Composés de formule :
A'-Dap-Fmoc
dans laquelle A' est une unité de formule A telle que définie ci-dessus, dont les fragments carboxy ou phosphoniques sont protégés de manière appropriée, Dap est un diaminoacide, spécifiquement l'acide 2,3-diaminopropionique et Fmoc est le (9H-fluorèn-9-ylméthoxy)carbonyle.

11. Composé selon la revendication 10, qui est la 3-[[(4*S*)-4-[bis[2-[bis[2-(1,1-diméthyléthoxy)-2-oxoéthyl]amino]éthyl]amino]-5-(1,1-diméthyléthoxy)-1,5-dioxopentyl]amino]-N-[(9*H*-fluorèn-9-ylméthoxy)carbonyl]-L-alanine.
